# EUROPEAN PATENT APPLICATION

(11) **EP 4 790 725 A2**
(43) Date of publication of application: **12.08.2026**
(21) Application number: 26188095.9
(22) Date of filing: 15.05.2024
(51) Int. Cl.: G16H 50/30

(54) **DETECTING EXERCISE ACTIVITY USING WIRELESS SIGNALS**

(30) Priority: 29.05.2023 GR 20230100427
(62) Document number(s) of the earlier application(s) in accordance with Art. 76 EPC: 24734274.4 / 4 721 090
(71) Applicant: QUALCOMM INCORPORATED, San Diego, California 92121-1714 (US)
(72) Inventor: REDDY, Varun Amar, San Diego, 92121 (US); MANOLAKOS, Alexandros, San Diego, 92121 (US)
(74) Representative: Reddie & Grose LLP

(57) **Abstract**

A set of UEs may be configured to cooperate to calculate an exercise state of a user of the set of UEs. A UE may obtain at least one of a set of relative ranges between UE pairs of a set of UEs, a set of relative angles between the UE pairs of the set of UEs, or a set of radio frequency (RF) sensing measurements associated with a user of the set of UEs. The UE may calculate an exercise state based on at least one of the set of relative ranges, the set of relative angles, or the set of RF sensing measurements.

## Description

### CROSS-REFERENCE TO RELATED APPLICATION

This application claims the benefit of Greek Patent Application Serial No. 20230100427, entitled "DETECTING EXERCISE ACTIVITY USING WIRELESS SIGNALS" and filed on May 29, 2023, which is expressly incorporated by reference herein in its entirety.

### TECHNICAL FIELD

The present disclosure relates generally to communication systems, and more particularly, to a wireless exercise monitoring system.

### INTRODUCTION

Wireless communication systems are widely deployed to provide various telecommunication services such as telephony, video, data, messaging, and broadcasts. Typical wireless communication systems may employ multiple-access technologies capable of supporting communication with multiple users by sharing available system resources. Examples of such multiple-access technologies include code division multiple access (CDMA) systems, time division multiple access (TDMA) systems, frequency division multiple access (FDMA) systems, orthogonal frequency division multiple access (OFDMA) systems, single-carrier frequency division multiple access (SC-FDMA) systems, and time division synchronous code division multiple access (TD-SCDMA) systems.

These multiple access technologies have been adopted in various telecommunication standards to provide a common protocol that enables different wireless devices to communicate on a municipal, national, regional, and even global level. An example telecommunication standard is 5G New Radio (NR). 5G NR is part of a continuous mobile broadband evolution promulgated by Third Generation Partnership Project (3GPP) to meet new requirements associated with latency, reliability, security, scalability (e.g., with Internet of Things (IoT)), and other requirements. 5G NR includes services associated with enhanced mobile broadband (eMBB), massive machine type communications (mMTC), and ultra-reliable low latency communications (URLLC). Some aspects of 5G NR may be based on the 4G Long Term Evolution (LTE) standard. There exists a need for further improvements in 5G NR technology. These improvements may also be applicable to other multi-access technologies and the telecommunication standards that employ these technologies.

### BRIEF SUMMARY

The following presents a simplified summary of one or more aspects in order to provide a basic understanding of such aspects. This summary is not an extensive overview of all contemplated aspects. This summary neither identifies key or critical elements of all aspects nor delineates the scope of any or all aspects. Its sole purpose is to present some concepts of one or more aspects in a simplified form as a prelude to the more detailed description that is presented later.

In an aspect of the disclosure, a method, a computer-readable medium, and an apparatus are provided. The apparatus may include a user equipment (UE). The apparatus may obtain at least one of a set of relative ranges between UE pairs of a set of UEs, a set of relative angles between the UE pairs of the set of UEs, or a set of radio frequency (RF) sensing measurements associated with a user of the set of UEs. The apparatus may calculate an exercise state based on at least one of the set of relative ranges, the set of relative angles, or the set of RF sensing measurements.

In an aspect of the disclosure, a method, a computer-readable medium, and an apparatus are provided. The apparatus may include a UE. The apparatus may receive a control message from at least one of a set of UEs. The apparatus may receive a set of positioning signals from at least one of the set of UEs based on the control message. The apparatus may measure the set of positioning signals based on the control message. The apparatus may calculate at least one of a set of relative ranges or a set of relative angles between the UE and at least one of the set of UEs based on the control message.

To the accomplishment of the foregoing and related ends, the one or more aspects may include the features hereinafter fully described and particularly pointed out in the claims. The following description and the drawings set forth in detail certain illustrative features of the one or more aspects. These features are indicative, however, of but a few of the various ways in which the principles of various aspects may be employed.

### BRIEF DESCRIPTION OF THE DRAWINGS

FIG. 1 is a diagram illustrating an example of a wireless communications system and an access network.
FIG. 2A is a diagram illustrating an example of a first frame, in accordance with various aspects of the present disclosure.
FIG. 2B is a diagram illustrating an example of downlink (DL) channels within a subframe, in accordance with various aspects of the present disclosure.
FIG. 2C is a diagram illustrating an example of a second frame, in accordance with various aspects of the present disclosure.
FIG. 2D is a diagram illustrating an example of uplink (UL) channels within a subframe, in accordance with various aspects of the present disclosure.
FIG. 3 is a diagram illustrating an example of a base station and user equipment (UE) in an access network.
FIG. 4 is a diagram illustrating an example of positioning based on positioning signal measurements.
FIG. 5 is a diagram illustrating an example of sensing based on sensing signal measurements.
FIG. 6 is a diagram illustrating a user wearing a set of UEs that may be used to calculate an exercise state of the user.
FIG. 7 is a connection flow diagram between UEs configured to calculate an exercise state of the user.
FIG. 8 is a connection flow diagram between UEs configured to calculate an exercise state of the user.
FIG. 9 is a flowchart of a method of wireless communication.
FIG. 10 is a flowchart of a method of wireless communication.
FIG. 11 is a flowchart of a method of wireless communication.
FIG. 12 is a flowchart of a method of wireless communication.
FIG. 13 is a flowchart of a method of wireless communication.
FIG. 14 is a flowchart of a method of wireless communication.
FIG. 15 is a flowchart of a method of wireless communication.
FIG. 16 is a diagram illustrating an example of a hardware implementation for an example apparatus and/or network entity.

### DETAILED DESCRIPTION

The following description is directed to examples for the purposes of describing innovative aspects of this disclosure. However, a person having ordinary skill in the art may recognize that the teachings herein may be applied in a multitude of ways. Some or all of the described examples may be implemented in any device, system or network that is capable of transmitting and receiving radio frequency (RF) signals according to one or more of the Institute of Electrical and Electronics Engineers (IEEE) 802.11 standards, the IEEE 802.15 standards, the Bluetooth^{®} standards as defined by the Bluetooth Special Interest Group (SIG), or the Long Term Evolution (LTE), 3G, 4G or 5G (New Radio (NR)) standards promulgated by the 3rd Generation Partnership Project (3GPP), among others. The described examples may be implemented in any device, system or network that is capable of transmitting and receiving RF signals according to one or more of the following technologies or techniques: code division multiple access (CDMA), time division multiple access (TDMA), frequency division multiple access (FDMA), orthogonal FDMA (OFDMA), single-carrier FDMA (SC-FDMA), spatial division multiple access (SDMA), rate-splitting multiple access (RSMA), multi-user shared access (MUSA), single-user (SU) multiple-input multiple-output (MIMO) and multi-user (MU)-MIMO. The described examples also may be implemented using other wireless communication protocols or RF signals suitable for use in one or more of a wireless personal area network (WPAN), a wireless local area network (WLAN), a wireless wide area network (WWAN), a wireless metropolitan area network (WMAN), or an internet of things (IoT) network.

Fitness devices, such as smart watches or smart rings, may have sensors that automatically detect whether a user of the fitness device has started working out. This may allow for users to accurately track the type of exercise that they perform. In some aspects, information from a sensor, such as an inertial measurement unit (IMU), may report translational motion and/or rotational motion of a user. Such information may be used to determine if the user has started exercising. A fitness device may use an exercise detection model to process such information to determine if the user has started exercising. Machine learning or pattern recognition methods may be used to build an exercise detection model based on inputs from such sensors. In some aspects, an exercise detection model may be generated using artificial intelligence machine learning (AI/ML) using a set of inputs from sensors and a set of labels. For example, a user may run, walk, and dance a fitness device, and the inputs from the fitness device may be used to train an exercise detection model with a first set of sensor information associated with the known action of running, a second set of sensor information associated with the known action of walking, and a third set of sensor information associated with the known action of dancing. Training may include a user exercising with the fitness device and manually entering what type of exercise the user is performing during a time period when the sensor information is recorded. Such a training phase may extend over a long period of time (e.g., hours) before the exercise detection model collects enough information to effectively differentiate between different exercise movements. The trained exercise detection model may then be used to determine what actions the user is taking based on sensor information from the fitness device. A trained exercise detection model may be able to detect a plurality of workout types, such as running, elliptical movements, or swimming.

Such fitness devices may have limited memory and computational capability. When such a device receives a software update or a firmware update, the exercise detection model may need to be retrained, as the method or format a sensor uses to collect information may change. In some aspects, one exercise detection model may be copied to a plurality of fitness devices so that the fitness devices may have a default exercise detection model applicable to a plurality of users. While such fitness devices may detect a plurality of workouts, the number and type of detectable workouts may be limited due to sensor limitations. Moreover, such limited sensor information may cause even a well-trained exercise detection model to detect exercises incorrectly. In some aspects, a fitness device may be improved by incorporating RF positioning and/or sensing technologies to detect the position of wireless devices relative to one another, or detect the position of reflective objects relative to a wireless device transmitting or receiving a sensing signal.

Various aspects relate generally to wireless communication and more particularly to wirelessly monitoring the exercise state of a user of a set of user equipment (UEs). Some aspects more specifically relate to monitoring the exercise state of a user of a set of UEs using wireless positioning and sensing using radio frequency (RF) signals. In some examples, a UE may obtain at least one of a set of relative ranges between UE pairs of a set of UEs, a set of relative angles between the UE pairs of the set of UEs, or a set of RF sensing measurements associated with a user of the set of UEs. The UE may calculate an exercise state based on at least one of the set of relative ranges, the set of relative angles, or the set of RF sensing measurements. In some examples, a UE may receive a control message from at least one of a set of UEs. The UE may receive a set of positioning signals from at least one of the set of UEs based on the control message. The UE may measure the set of positioning signals based on the control message. The UE may calculate at least one of a set of relative ranges or a set of relative angles between the UE and at least one of the set of UEs based on the control message.

The disclosed UEs may improve the accuracy and efficiency of exercise tracking devices like a smart watch or a smart ring using RF signals, such as ultra-wide band (UWB) signals, Bluetooth low energy (BLE) signals or Wi-Fi signals. Such signals may be measured to detect the relative position of wireless devices with respect to one another, and/or to detect the relative position of reflective target objects relative to wireless devices that transmit or receive sensing signals. In some aspects, an exercise detection model may be trained using a combination of RF measurements and other measurements, such as IMU measurements or global navigation satellite system (GNSS) measurements. In some aspects, an exercise detection model may indicate whether a user is resting, has begun exercising, the type of exercise being performed by the user, and/or whether the user is performing the exercise correctly, based on inputs to the exercise detection model. The inputs may include a relative range between all pairs of devices, for example measured using a time of arrival (ToA). The inputs may include a relative angle between pairs of devices, for example measured by calculating/estimating an angle of arrival (AoA) of signals received from other UEs. The inputs may include sensing measurements made between pairs of devices, for example using channel impulse response (CIR) measurements. A UE may include any device capable of transmitting a positioning signal and/or a sensing signal. A UE may include any device capable of measuring a positioning signal and/or a reflected sensing signal. A UE may include earbuds, a smartphone, a smart watch, and/or a smart ring. The exercise detection model may be contained by any such UE. In one aspect, a user's mobility information may be inferred based on periodic patterns of relative ranges between different pairs of devices. In one aspect, to detect relative ranges between different pairs of devices, UWB may be used. In one aspect, a response message (e.g., a ranging response message (RRM)) from a responder may include inertial measurement unit (IMU) data of the responder. In one aspect, the periodicity of UWB ranging may be updated through a control message (e.g., a ranging control message (RCM) or a ranging control update message (RCUM)) from an initiator to a responder based on IMU information from different devices pointing to rapid user movement. In one aspect, a smart device (like smart watch or smart ring) of the user may collect and process data, to train a model that is in turn used to detect exercise activity. In one aspect, the smart device may offload such computations to a detection model in a smartphone of the user, or a remote server, wherein the detection model may be developed using crowdsourced data from various users. In one aspect, different detection models may be developed based on attributes of different body types (height, weight, etc). In one aspect, a smartphone may act as a UWB initiator and receive relative ranges and angles between all other smart devices which may act as responders. In one aspect, fitness equipment like a treadmill, elliptical, bike, etc. could act as a UWB initiator and display statistics and cues on a screen for the user. In one aspect, the exercise equipment may also include Wi-Fi, BLE, and/or monostatic sensing measurements along with UWB to develop a second detection model. The second detection model may also crowdsource data from various users of the exercise equipment. In one aspect, access points (Wi-Fi/UWB/BLE) can also be used to make additional measurements with the smart devices that are equipped by the user to develop a second detection model.

Particular aspects of the subject matter described in this disclosure can be implemented to realize one or more of the following potential advantages. In some examples, by configuring a UE to calculate an exercise state or a user based on at least one of a set of relative ranges, a set of relative angles, or a set of RF sensing measurements received by one or more UEs of a set of UEs, the described techniques can be used to accurately detect the exercise of a user based on a plurality of measurements about the body of an exercising user instead of at just one or two points at the body (e.g., a wrist of a smartwatch or a dongle attached to an arm or a waistband of an exercising user). The UE may be trained to detect a variety of exercises instead of just one or two exercises because of the plethora of data inputs to the exercise detection device.

The detailed description set forth below in connection with the drawings describes various configurations and does not represent the only configurations in which the concepts described herein may be practiced. The detailed description includes specific details for the purpose of providing a thorough understanding of various concepts. However, these concepts may be practiced without these specific details. In some instances, well known structures and components are shown in block diagram form in order to avoid obscuring such concepts.

Several aspects of telecommunication systems are presented with reference to various apparatus and methods. These apparatus and methods are described in the following detailed description and illustrated in the accompanying drawings by various blocks, components, circuits, processes, algorithms, etc. (collectively referred to as "elements"). These elements may be implemented using electronic hardware, computer software, or any combination thereof. Whether such elements are implemented as hardware or software depends upon the particular application and design constraints imposed on the overall system.

By way of example, an element, or any portion of an element, or any combination of elements may be implemented as a "processing system" that includes one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure. One or more processors in the processing system may execute software. Software, whether referred to as software, firmware, middleware, microcode, hardware description language, or otherwise, shall be construed broadly to mean instructions, instruction sets, code, code segments, program code, programs, subprograms, software components, applications, software applications, software packages, routines, subroutines, objects, executables, threads of execution, procedures, functions, or any combination thereof.

Accordingly, in one or more example aspects, implementations, and/or use cases, the functions described may be implemented in hardware, software, or any combination thereof. If implemented in software, the functions may be stored on or encoded as one or more instructions or code on a computer-readable medium. Computer-readable media includes computer storage media. Storage media may be any available media that can be accessed by a computer. By way of example, such computer-readable media can include a random-access memory (RAM), a read-only memory (ROM), an electrically erasable programmable ROM (EEPROM), optical disk storage, magnetic disk storage, other magnetic storage devices, combinations of the types of computer-readable media, or any other medium that can be used to store computer executable code in the form of instructions or data structures that can be accessed by a computer.

While aspects, implementations, and/or use cases are described in this application by illustration to some examples, additional or different aspects, implementations and/or use cases may come about in many different arrangements and scenarios. Aspects, implementations, and/or use cases described herein may be implemented across many differing platform types, devices, systems, shapes, sizes, and packaging arrangements. For example, aspects, implementations, and/or use cases may come about via integrated chip implementations and other non-module-component based devices (e.g., end-user devices, vehicles, communication devices, computing devices, industrial equipment, retail/purchasing devices, medical devices, artificial intelligence (Al)-enabled devices, etc.). While some examples may or may not be specifically directed to use cases or applications, a wide assortment of applicability of described examples may occur. Aspects, implementations, and/or use cases may range a spectrum from chip-level or modular components to non-modular, non-chip-level implementations and further to aggregate, distributed, or original equipment manufacturer (OEM) devices or systems incorporating one or more techniques herein. In some practical settings, devices incorporating described aspects and features may also include additional components and features for implementation and practice of claimed and described aspect. For example, transmission and reception of wireless signals necessarily includes a number of components for analog and digital purposes (e.g., hardware components including antenna, RF-chains, power amplifiers, modulators, buffer, processor(s), interleaver, adders/summers, etc.). Techniques described herein may be practiced in a wide variety of devices, chip-level components, systems, distributed arrangements, aggregated or disaggregated components, end-user devices, etc. of varying sizes, shapes, and constitution.

Deployment of communication systems, such as 5G NR systems, may be arranged in multiple manners with various components or constituent parts. In a 5G NR system, or network, a network node, a network entity, a mobility element of a network, a radio access network (RAN) node, a core network node, a network element, or a network equipment, such as a base station (BS), or one or more units (or one or more components) performing base station functionality, may be implemented in an aggregated or disaggregated architecture. For example, a BS (such as a Node B (NB), evolved NB (eNB), NR BS, 5G NB, access point (AP), a transmission reception point (TRP), or a cell, etc.) may be implemented as an aggregated base station (also known as a standalone BS or a monolithic BS) or a disaggregated base station.

An aggregated base station may be configured to utilize a radio protocol stack that is physically or logically integrated within a single RAN node. A disaggregated base station may be configured to utilize a protocol stack that is physically or logically distributed among two or more units (such as one or more central or centralized units (CUs), one or more distributed units (DUs), or one or more radio units (RUs)). In some aspects, a CU may be implemented within a RAN node, and one or more DUs may be co-located with the CU, or alternatively, may be geographically or virtually distributed throughout one or multiple other RAN nodes. The DUs may be implemented to communicate with one or more RUs. Each of the CU, DU and RU can be implemented as virtual units, i.e., a virtual central unit (VCU), a virtual distributed unit (VDU), or a virtual radio unit (VRU).

Base station operation or network design may consider aggregation characteristics of base station functionality. For example, disaggregated base stations may be utilized in an integrated access backhaul (IAB) network, an open radio access network (O-RAN (such as the network configuration sponsored by the O-RAN Alliance)), or a virtualized radio access network (vRAN, also known as a cloud radio access network (C-RAN)). Disaggregation may include distributing functionality across two or more units at various physical locations, as well as distributing functionality for at least one unit virtually, which can enable flexibility in network design. The various units of the disaggregated base station, or disaggregated RAN architecture, can be configured for wired or wireless communication with at least one other unit.

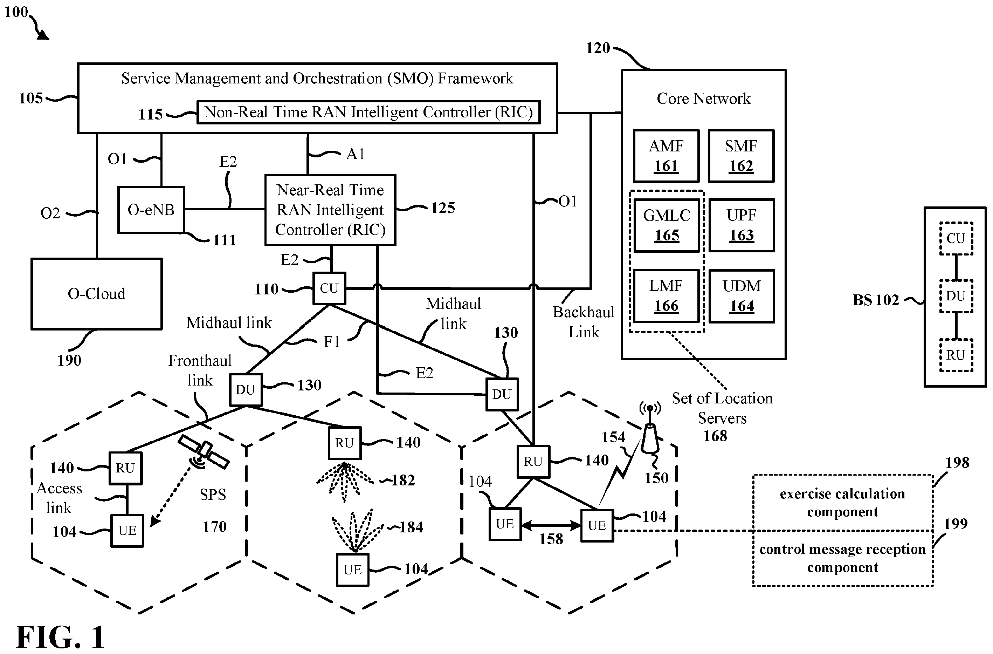

FIG. 1 is a diagram 100 illustrating an example of a wireless communications system and an access network. The illustrated wireless communications system includes a disaggregated base station architecture. The disaggregated base station architecture may include one or more CUs 110 that can communicate directly with a core network 120 via a backhaul link, or indirectly with the core network 120 through one or more disaggregated base station units (such as a Near-Real Time (Near-RT) RAN Intelligent Controller (RIC) 125 via an E2 link, or a Non-Real Time (Non-RT) RIC 115 associated with a Service Management and Orchestration (SMO) Framework 105, or both). A CU 110 may communicate with one or more DUs 130 via respective midhaul links, such as an F1 interface. The DUs 130 may communicate with one or more RUs 140 via respective fronthaul links. The RUs 140 may communicate with respective UEs 104 via one or more radio frequency (RF) access links. In some implementations, the UE 104 may be simultaneously served by multiple RUs 140.

Each of the units, i.e., the CUs 110, the DUs 130, the RUs 140, as well as the Near-RT RICs 125, the Non-RT RICs 115, and the SMO Framework 105, may include one or more interfaces or be coupled to one or more interfaces configured to receive or to transmit signals, data, or information (collectively, signals) via a wired or wireless transmission medium. Each of the units, or an associated processor or controller providing instructions to the communication interfaces of the units, can be configured to communicate with one or more of the other units via the transmission medium. For example, the units can include a wired interface configured to receive or to transmit signals over a wired transmission medium to one or more of the other units. Additionally, the units can include a wireless interface, which may include a receiver, a transmitter, or a transceiver (such as an RF transceiver), configured to receive or to transmit signals, or both, over a wireless transmission medium to one or more of the other units.

In some aspects, the CU 110 may host one or more higher layer control functions. Such control functions can include radio resource control (RRC), packet data convergence protocol (PDCP), service data adaptation protocol (SDAP), or the like. Each control function can be implemented with an interface configured to communicate signals with other control functions hosted by the CU 110. The CU 110 may be configured to handle user plane functionality (i.e., Central Unit - User Plane (CU-UP)), control plane functionality (i.e., Central Unit - Control Plane (CU-CP)), or a combination thereof. In some implementations, the CU 110 can be logically split into one or more CU-UP units and one or more CU-CP units. The CU-UP unit can communicate bidirectionally with the CU-CP unit via an interface, such as an E1 interface when implemented in an O-RAN configuration. The CU 110 can be implemented to communicate with the DU 130, as necessary, for network control and signaling.

The DU 130 may correspond to a logical unit that includes one or more base station functions to control the operation of one or more RUs 140. In some aspects, the DU 130 may host one or more of a radio link control (RLC) layer, a medium access control (MAC) layer, and one or more high physical (PHY) layers (such as modules for forward error correction (FEC) encoding and decoding, scrambling, modulation, demodulation, or the like) depending, at least in part, on a functional split, such as those defined by 3GPP. In some aspects, the DU 130 may further host one or more low PHY layers. Each layer (or module) can be implemented with an interface configured to communicate signals with other layers (and modules) hosted by the DU 130, or with the control functions hosted by the CU 110.

Lower-layer functionality can be implemented by one or more RUs 140. In some deployments, an RU 140, controlled by a DU 130, may correspond to a logical node that hosts RF processing functions, or low-PHY layer functions (such as performing fast Fourier transform (FFT), inverse FFT (iFFT), digital beamforming, physical random access channel (PRACH) extraction and filtering, or the like), or both, based at least in part on the functional split, such as a lower layer functional split. In such an architecture, the RU(s) 140 can be implemented to handle over the air (OTA) communication with one or more UEs 104. In some implementations, real-time and non-real-time aspects of control and user plane communication with the RU(s) 140 can be controlled by the corresponding DU 130. In some scenarios, this configuration can enable the DU(s) 130 and the CU 110 to be implemented in a cloud-based RAN architecture, such as a vRAN architecture.

The SMO Framework 105 may be configured to support RAN deployment and provisioning of non-virtualized and virtualized network elements. For non-virtualized network elements, the SMO Framework 105 may be configured to support the deployment of dedicated physical resources for RAN coverage requirements that may be managed via an operations and maintenance interface (such as an O1 interface). For virtualized network elements, the SMO Framework 105 may be configured to interact with a cloud computing platform (such as an open cloud (O-Cloud) 190) to perform network element life cycle management (such as to instantiate virtualized network elements) via a cloud computing platform interface (such as an O2 interface). Such virtualized network elements can include, but are not limited to, CUs 110, DUs 130, RUs 140 and Near-RT RICs 125. In some implementations, the SMO Framework 105 can communicate with a hardware aspect of a 4G RAN, such as an open eNB (O-eNB) 111, via an O1 interface. Additionally, in some implementations, the SMO Framework 105 can communicate directly with one or more RUs 140 via an O1 interface. The SMO Framework 105 also may include a Non-RT RIC 115 configured to support functionality of the SMO Framework 105.

The Non-RT RIC 115 may be configured to include a logical function that enables non-real-time control and optimization of RAN elements and resources, artificial intelligence (AI) / machine learning (ML) (AI/ML) workflows including model training and updates, or policy-based guidance of applications/features in the Near-RT RIC 125. The Non-RT RIC 115 may be coupled to or communicate with (such as via an Al interface) the Near-RT RIC 125. The Near-RT RIC 125 may be configured to include a logical function that enables near-real-time control and optimization of RAN elements and resources via data collection and actions over an interface (such as via an E2 interface) connecting one or more CUs 110, one or more DUs 130, or both, as well as an O-eNB, with the Near-RT RIC 125.

In some implementations, to generate AI/ML models to be deployed in the Near-RT RIC 125, the Non-RT RIC 115 may receive parameters or external enrichment information from external servers. Such information may be utilized by the Near-RT RIC 125 and may be received at the SMO Framework 105 or the Non-RT RIC 115 from non-network data sources or from network functions. In some examples, the Non-RT RIC 115 or the Near-RT RIC 125 may be configured to tune RAN behavior or performance. For example, the Non-RT RIC 115 may monitor long-term trends and patterns for performance and employ AI/ML models to perform corrective actions through the SMO Framework 105 (such as reconfiguration via O1) or via creation of RAN management policies (such as A1 policies).

At least one of the CU 110, the DU 130, and the RU 140 may be referred to as a base station 102. Accordingly, a base station 102 may include one or more of the CU 110, the DU 130, and the RU 140 (each component indicated with dotted lines to signify that each component may or may not be included in the base station 102). The base station 102 provides an access point to the core network 120 for a UE 104. The base station 102 may include macrocells (high power cellular base station) and/or small cells (low power cellular base station). The small cells include femtocells, picocells, and microcells. A network that includes both small cell and macrocells may be known as a heterogeneous network. A heterogeneous network may also include Home Evolved Node Bs (eNBs) (HeNBs), which may provide service to a restricted group known as a closed subscriber group (CSG). The communication links between the RUs 140 and the UEs 104 may include uplink (UL) (also referred to as reverse link) transmissions from a UE 104 to an RU 140 and/or downlink (DL) (also referred to as forward link) transmissions from an RU 140 to a UE 104. The communication links may use multiple-input and multiple-output (MIMO) antenna technology, including spatial multiplexing, beamforming, and/or transmit diversity. The communication links may be through one or more carriers. The base station 102 / UEs 104 may use spectrum up to *YMHz* (e.g., 5, 10, 15, 20, 100, 400, etc. MHz) bandwidth per carrier allocated in a carrier aggregation of up to a total of *Yx* MHz (x component carriers) used for transmission in each direction. The carriers may or may not be adjacent to each other. Allocation of carriers may be asymmetric with respect to DL and UL (e.g., more or fewer carriers may be allocated for DL than for UL). The component carriers may include a primary component carrier and one or more secondary component carriers. A primary component carrier may be referred to as a primary cell (PCell) and a secondary component carrier may be referred to as a secondary cell (SCell).

Certain UEs 104 may communicate with each other using device-to-device (D2D) communication link 158. The D2D communication link 158 may use the DL/UL wireless wide area network (WWAN) spectrum. The D2D communication link 158 may use one or more sidelink channels, such as a physical sidelink broadcast channel (PSBCH), a physical sidelink discovery channel (PSDCH), a physical sidelink shared channel (PSSCH), and a physical sidelink control channel (PSCCH). D2D communication may be through a variety of wireless D2D communications systems, such as for example, Bluetooth, Wi-Fi based on the Institute of Electrical and Electronics Engineers (IEEE) 802.11 standard, LTE, or NR.

The wireless communications system may further include a Wi-Fi AP 150 in communication with UEs 104 (also referred to as Wi-Fi stations (STAs)) via communication link 154, e.g., in a 5 GHz unlicensed frequency spectrum or the like. When communicating in an unlicensed frequency spectrum, the UEs 104 / AP 150 may perform a clear channel assessment (CCA) prior to communicating in order to determine whether the channel is available.

The electromagnetic spectrum is often subdivided, based on frequency/wavelength, into various classes, bands, channels, etc. In 5G NR, two initial operating bands have been identified as frequency range designations FR1 (410 MHz - 7.125 GHz) and FR2 (24.25 GHz - 52.6 GHz). Although a portion of FR1 is greater than 6 GHz, FR1 is often referred to (interchangeably) as a "sub-6 GHz" band in various documents and articles. A similar nomenclature issue sometimes occurs with regard to FR2, which is often referred to (interchangeably) as a "millimeter wave" band in documents and articles, despite being different from the extremely high frequency (EHF) band (30 GHz - 300 GHz) which is identified by the International Telecommunications Union (ITU) as a "millimeter wave" band.

The frequencies between FR1 and FR2 are often referred to as mid-band frequencies. Recent 5G NR studies have identified an operating band for these mid-band frequencies as frequency range designation FR3 (7.125 GHz - 24.25 GHz). Frequency bands falling within FR3 may inherit FR1 characteristics and/or FR2 characteristics, and thus may effectively extend features of FR1 and/or FR2 into mid-band frequencies. In addition, higher frequency bands are currently being explored to extend 5G NR operation beyond 52.6 GHz. For example, three higher operating bands have been identified as frequency range designations FR2-2 (52.6 GHz - 71 GHz), FR4 (71 GHz - 114.25 GHz), and FR5 (114.25 GHz- 300 GHz). Each of these higher frequency bands falls within the EHF band.

With the above aspects in mind, unless specifically stated otherwise, the term "sub-6 GHz" or the like if used herein may broadly represent frequencies that may be less than 6 GHz, may be within FR1, or may include mid-band frequencies. Further, unless specifically stated otherwise, the term "millimeter wave" or the like if used herein may broadly represent frequencies that may include mid-band frequencies, may be within FR2, FR4, FR2-2, and/or FR5, or may be within the EHF band.

The base station 102 and the UE 104 may each include a plurality of antennas, such as antenna elements, antenna panels, and/or antenna arrays to facilitate beamforming. The base station 102 may transmit a beamformed signal 182 to the UE 104 in one or more transmit directions. The UE 104 may receive the beamformed signal from the base station 102 in one or more receive directions. The UE 104 may also transmit a beamformed signal 184 to the base station 102 in one or more transmit directions. The base station 102 may receive the beamformed signal from the UE 104 in one or more receive directions. The base station 102 / UE 104 may perform beam training to determine the best receive and transmit directions for each of the base station 102 / UE 104. The transmit and receive directions for the base station 102 may or may not be the same. The transmit and receive directions for the UE 104 may or may not be the same.

The base station 102 may include and/or be referred to as a gNB, Node B, eNB, an access point, a base transceiver station, a radio base station, a radio transceiver, a transceiver function, a basic service set (BSS), an extended service set (ESS), a TRP, network node, network entity, network equipment, or some other suitable terminology. The base station 102 can be implemented as an integrated access and backhaul (IAB) node, a relay node, a sidelink node, an aggregated (monolithic) base station with a baseband unit (BBU) (including a CU and a DU) and an RU, or as a disaggregated base station including one or more of a CU, a DU, and/or an RU. The set of base stations, which may include disaggregated base stations and/or aggregated base stations, may be referred to as next generation (NG) RAN (NG-RAN).

The core network 120 may include an Access and Mobility Management Function (AMF) 161, a Session Management Function (SMF) 162, a User Plane Function (UPF) 163, a Unified Data Management (UDM) 164, one or more location servers 168, and other functional entities. The AMF 161 is the control node that processes the signaling between the UEs 104 and the core network 120. The AMF 161 supports registration management, connection management, mobility management, and other functions. The SMF 162 supports session management and other functions. The UPF 163 supports packet routing, packet forwarding, and other functions. The UDM 164 supports the generation of authentication and key agreement (AKA) credentials, user identification handling, access authorization, and subscription management. The one or more location servers 168 are illustrated as including a Gateway Mobile Location Center (GMLC) 165 and a Location Management Function (LMF) 166. However, generally, the one or more location servers 168 may include one or more location/positioning servers, which may include one or more of the GMLC 165, the LMF 166, a position determination entity (PDE), a serving mobile location center (SMLC), a mobile positioning center (MPC), or the like. The GMLC 165 and the LMF 166 support UE location services. The GMLC 165 provides an interface for clients/applications (e.g., emergency services) for accessing UE positioning information. The LMF 166 receives measurements and assistance information from the NG-RAN and the UE 104 via the AMF 161 to compute the position of the UE 104. The NG-RAN may utilize one or more positioning methods in order to determine the position of the UE 104. Positioning the UE 104 may involve signal measurements, a position estimate, and an optional velocity computation based on the measurements. The signal measurements may be made by the UE 104 and/or the base station 102 serving the UE 104. The signals measured may be based on one or more of a satellite positioning system (SPS) 170 (e.g., one or more of a Global Navigation Satellite System (GNSS), global position system (GPS), non-terrestrial network (NTN), or other satellite position/location system), LTE signals, wireless local area network (WLAN) signals, Bluetooth signals, a terrestrial beacon system (TBS), sensor-based information (e.g., barometric pressure sensor, motion sensor), NR enhanced cell ID (NR E-CID) methods, NR signals (e.g., multi-round trip time (Multi-RTT), DL angle-of-departure (DL-AoD), DL time difference of arrival (DL-TDOA), UL time difference of arrival (UL-TDOA), and UL angle-of-arrival (UL-AoA) positioning), and/or other systems/signals/sensors.

Examples of UEs 104 include a cellular phone, a smart phone, a session initiation protocol (SIP) phone, a laptop, a personal digital assistant (PDA), a satellite radio, a global positioning system, a multimedia device, a video device, a digital audio player (e.g., MP3 player), a camera, a game console, a tablet, a smart device, a wearable device, a vehicle, an electric meter, a gas pump, a large or small kitchen appliance, a healthcare device, an implant, a sensor/actuator, a display, or any other similar functioning device. Some of the UEs 104 may be referred to as IoT devices (e.g., parking meter, gas pump, toaster, vehicles, heart monitor, etc.). The UE 104 may also be referred to as a station, a mobile station, a subscriber station, a mobile unit, a subscriber unit, a wireless unit, a remote unit, a mobile device, a wireless device, a wireless communications device, a remote device, a mobile subscriber station, an access terminal, a mobile terminal, a wireless terminal, a remote terminal, a handset, a user agent, a mobile client, a client, or some other suitable terminology. In some scenarios, the term UE may also apply to one or more companion devices such as in a device constellation arrangement. One or more of these devices may collectively access the network and/or individually access the network.

Referring again to FIG. 1, in certain aspects, the UE 104 may have an exercise calculation component 198 that may be configured to obtain at least one of a set of relative ranges between UE pairs of a set of UEs, a set of relative angles between the UE pairs of the set of UEs, or a set of RF sensing measurements associated with a user of the set of UEs. The exercise calculation component 198 may be configured to calculate an exercise state based on at least one of the set of relative ranges, the set of relative angles, or the set of RF sensing measurements. In certain aspects, the UE 104 may have a control message reception component 199 that may be configured to receive a control message from at least one of a set of UEs. The control message reception component 199 may be configured to receive a set of positioning signals from at least one of the set of UEs based on the control message. The control message reception component 199 may be configured to measure the set of positioning signals based on the control message. The control message reception component 199 may be configured to calculate at least one of a set of relative ranges or a set of relative angles between the UE and at least one of the set of UEs based on the control message. A user may wear the set of UEs while exercising. While the user exercises, the UEs may perform positioning relative to one another, and/or may perform sensing on parts of the user's body or objects worn by the user, such as other UEs or clothing worn by the user. A UE may use the positioning and/or sensing to calculate an exercise state of the user, for example detecting that the user is walking, running, resting, or performing an exercise in a non-optimal way that may be improved by a suggestion from a UE.

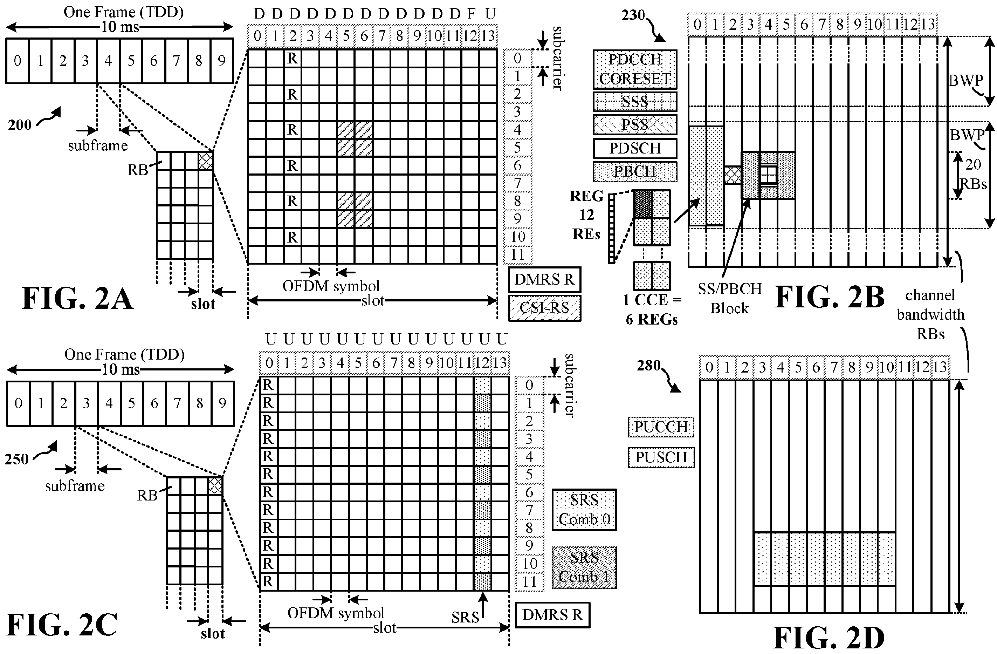

FIG. 2A is a diagram 200 illustrating an example of a first subframe within a 5G NR frame structure. FIG. 2B is a diagram 230 illustrating an example of DL channels within a 5G NR subframe. FIG. 2C is a diagram 250 illustrating an example of a second subframe within a 5G NR frame structure. FIG. 2D is a diagram 280 illustrating an example of UL channels within a 5G NR subframe. The 5G NR frame structure may be frequency division duplexed (FDD) in which for a particular set of subcarriers (carrier system bandwidth), subframes within the set of subcarriers are dedicated for either DL or UL, or may be time division duplexed (TDD) in which for a particular set of subcarriers (carrier system bandwidth), subframes within the set of subcarriers are dedicated for both DL and UL. In the examples provided by FIGs. 2A, 2C, the 5G NR frame structure is assumed to be TDD, with subframe 4 being configured with slot format 28 (with mostly DL), where D is DL, U is UL, and F is flexible for use between DL/UL, and subframe 3 being configured with slot format 1 (with all UL). While subframes 3, 4 are shown with slot formats 1, 28, respectively, any particular subframe may be configured with any of the various available slot formats 0-61. Slot formats 0, 1 are all DL, UL, respectively. Other slot formats 2-61 include a mix of DL, UL, and flexible symbols. UEs are configured with the slot format (dynamically through DL control information (DCI), or semi-statically/statically through radio resource control (RRC) signaling) through a received slot format indicator (SFI). Note that the description *infra* applies also to a 5G NR frame structure that is TDD.

FIGs. 2A-2D illustrate a frame structure, and the aspects of the present disclosure may be applicable to other wireless communication technologies, which may have a different frame structure and/or different channels. A frame (10 ms) may be divided into 10 equally sized subframes (1 ms). Each subframe may include one or more time slots. Subframes may also include mini-slots, which may include 7, 4, or 2 symbols. Each slot may include 14 or 12 symbols, depending on whether the cyclic prefix (CP) is normal or extended. For normal CP, each slot may include 14 symbols, and for extended CP, each slot may include 12 symbols. The symbols on DL may be CP orthogonal frequency division multiplexing (OFDM) (CP-OFDM) symbols. The symbols on UL may be CP-OFDM symbols (for high throughput scenarios) or discrete Fourier transform (DFT) spread OFDM (DFT-s-OFDM) symbols (for power limited scenarios; limited to a single stream transmission). The number of slots within a subframe is based on the CP and the numerology. The numerology defines the subcarrier spacing (SCS) (see Table 1). The symbol length/duration may scale with 1/SCS.

**Table 1: Numerology, SCS, and CP**

| **µ** | **SCS Δ*f* = 2^{µ} · 15 [kHz]** | **Cyclic prefix** |
|---|---|---|
| 0 | 15 | Normal |
| 1 | 30 | Normal |
| 2 | 60 | Normal, Extended |
| 3 | 120 | Normal |
| 4 | 240 | Normal |
| 5 | 480 | Normal |
| 6 | 960 | Normal |

For normal CP (14 symbols/slot), different numerologies µ 0 to 4 allow for 1, 2, 4, 8, and 16 slots, respectively, per subframe. For extended CP, the numerology 2 allows for 4 slots per subframe. Accordingly, for normal CP and numerology µ, there are 14 symbols/slot and 2^{µ} slots/subframe. The subcarrier spacing may be equal to 2*^{µ}* * 15 kHz, where *µ* is the numerology 0 to 4. As such, the numerology µ=0 has a subcarrier spacing of 15 kHz and the numerology µ=4 has a subcarrier spacing of 240 kHz. The symbol length/duration is inversely related to the subcarrier spacing. FIGs. 2A-2D provide an example of normal CP with 14 symbols per slot and numerology µ=2 with 4 slots per subframe. The slot duration is 0.25 ms, the subcarrier spacing is 60 kHz, and the symbol duration is approximately 16.67 µs. Within a set of frames, there may be one or more different bandwidth parts (BWPs) (see FIG. 2B) that are frequency division multiplexed. Each BWP may have a particular numerology and CP (normal or extended).

A resource grid may be used to represent the frame structure. Each time slot includes a resource block (RB) (also referred to as physical RBs (PRBs)) that extends 12 consecutive subcarriers. The resource grid is divided into multiple resource elements (REs). The number of bits carried by each RE depends on the modulation scheme.

As illustrated in FIG. 2A, some of the REs carry reference (pilot) signals (RS) for the UE. The RS may include demodulation RS (DM-RS) (indicated as R for one particular configuration, but other DM-RS configurations are possible) and channel state information reference signals (CSI-RS) for channel estimation at the UE. The RS may also include beam measurement RS (BRS), beam refinement RS (BRRS), and phase tracking RS (PT-RS).

FIG. 2B illustrates an example of various DL channels within a subframe of a frame. The physical downlink control channel (PDCCH) carries DCI within one or more control channel elements (CCEs) (e.g., 1, 2, 4, 8, or 16 CCEs), each CCE including six RE groups (REGs), each REG including 12 consecutive REs in an OFDM symbol of an RB. A PDCCH within one BWP may be referred to as a control resource set (CORESET). A UE is configured to monitor PDCCH candidates in a PDCCH search space (e.g., common search space, UE-specific search space) during PDCCH monitoring occasions on the CORESET, where the PDCCH candidates have different DCI formats and different aggregation levels. Additional BWPs may be located at greater and/or lower frequencies across the channel bandwidth. A primary synchronization signal (PSS) may be within symbol 2 of particular subframes of a frame. The PSS is used by a UE 104 to determine subframe/symbol timing and a physical layer identity. A secondary synchronization signal (SSS) may be within symbol 4 of particular subframes of a frame. The SSS is used by a UE to determine a physical layer cell identity group number and radio frame timing. Based on the physical layer identity and the physical layer cell identity group number, the UE can determine a physical cell identifier (PCI). Based on the PCI, the UE can determine the locations of the DM-RS. The physical broadcast channel (PBCH), which carries a master information block (MIB), may be logically grouped with the PSS and SSS to form a synchronization signal (SS)/PBCH block (also referred to as SS block (SSB)). The MIB provides a number of RBs in the system bandwidth and a system frame number (SFN). The physical downlink shared channel (PDSCH) carries user data, broadcast system information not transmitted through the PBCH such as system information blocks (SIBs), and paging messages.

As illustrated in FIG. 2C, some of the REs carry DM-RS (indicated as R for one particular configuration, but other DM-RS configurations are possible) for channel estimation at the base station. The UE may transmit DM-RS for the physical uplink control channel (PUCCH) and DM-RS for the physical uplink shared channel (PUSCH). The PUSCH DM-RS may be transmitted in the first one or two symbols of the PUSCH. The PUCCH DM-RS may be transmitted in different configurations depending on whether short or long PUCCHs are transmitted and depending on the particular PUCCH format used. The UE may transmit sounding reference signals (SRS). The SRS may be transmitted in the last symbol of a subframe. The SRS may have a comb structure, and a UE may transmit SRS on one of the combs. The SRS may be used by a base station for channel quality estimation to enable frequency-dependent scheduling on the UL.

FIG. 2D illustrates an example of various UL channels within a subframe of a frame. The PUCCH may be located as indicated in one configuration. The PUCCH carries uplink control information (UCI), such as scheduling requests, a channel quality indicator (CQI), a precoding matrix indicator (PMI), a rank indicator (RI), and hybrid automatic repeat request (HARQ) acknowledgment (ACK) (HARQ-ACK) feedback (i.e., one or more HARQ ACK bits indicating one or more ACK and/or negative ACK (NACK)). The PUSCH carries data, and may additionally be used to carry a buffer status report (BSR), a power headroom report (PHR), and/or UCI.

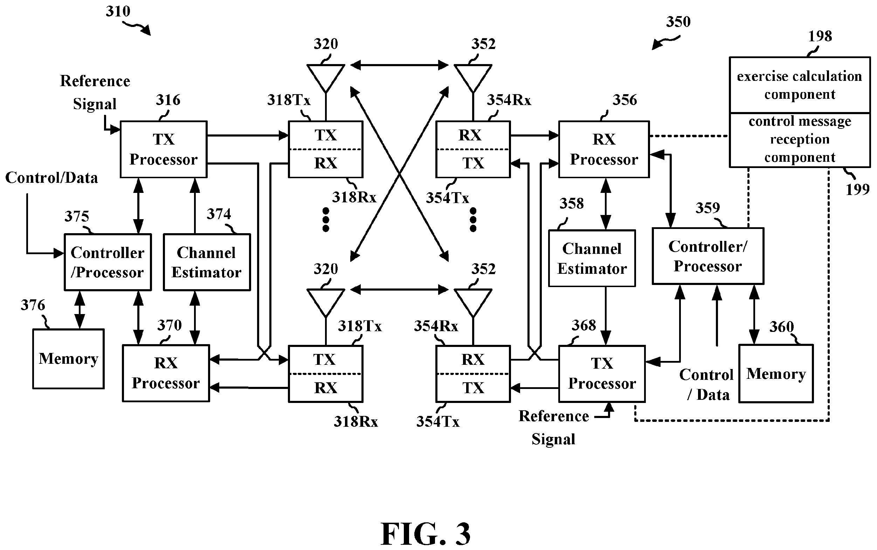

FIG. 3 is a block diagram of a base station 310 in communication with a UE 350 in an access network. In the DL, Internet protocol (IP) packets may be provided to a controller/processor 375. The controller/processor 375 implements layer 3 and layer 2 functionality. Layer 3 includes a radio resource control (RRC) layer, and layer 2 includes a service data adaptation protocol (SDAP) layer, a packet data convergence protocol (PDCP) layer, a radio link control (RLC) layer, and a medium access control (MAC) layer. The controller/processor 375 provides RRC layer functionality associated with broadcasting of system information (e.g., MIB, SIBs), RRC connection control (e.g., RRC connection paging, RRC connection establishment, RRC connection modification, and RRC connection release), inter radio access technology (RAT) mobility, and measurement configuration for UE measurement reporting; PDCP layer functionality associated with header compression / decompression, security (ciphering, deciphering, integrity protection, integrity verification), and handover support functions; RLC layer functionality associated with the transfer of upper layer packet data units (PDUs), error correction through ARQ, concatenation, segmentation, and reassembly of RLC service data units (SDUs), re-segmentation of RLC data PDUs, and reordering of RLC data PDUs; and MAC layer functionality associated with mapping between logical channels and transport channels, multiplexing of MAC SDUs onto transport blocks (TBs), demultiplexing of MAC SDUs from TBs, scheduling information reporting, error correction through HARQ, priority handling, and logical channel prioritization.

The transmit (TX) processor 316 and the receive (RX) processor 370 implement layer 1 functionality associated with various signal processing functions. Layer 1, which includes a physical (PHY) layer, may include error detection on the transport channels, forward error correction (FEC) coding/decoding of the transport channels, interleaving, rate matching, mapping onto physical channels, modulation/demodulation of physical channels, and MIMO antenna processing. The TX processor 316 handles mapping to signal constellations based on various modulation schemes (e.g., binary phase-shift keying (BPSK), quadrature phase-shift keying (QPSK), M-phase-shift keying (M-PSK), M-quadrature amplitude modulation (M-QAM)). The coded and modulated symbols may then be split into parallel streams. Each stream may then be mapped to an OFDM subcarrier, multiplexed with a reference signal (e.g., pilot) in the time and/or frequency domain, and then combined together using an Inverse Fast Fourier Transform (IFFT) to produce a physical channel carrying a time domain OFDM symbol stream. The OFDM stream is spatially precoded to produce multiple spatial streams. Channel estimates from a channel estimator 374 may be used to determine the coding and modulation scheme, as well as for spatial processing. The channel estimate may be derived from a reference signal and/or channel condition feedback transmitted by the UE 350. Each spatial stream may then be provided to a different antenna 320 via a separate transmitter 318Tx. Each transmitter 318Tx may modulate a radio frequency (RF) carrier with a respective spatial stream for transmission.

At the UE 350, each receiver 354Rx receives a signal through its respective antenna 352. Each receiver 354Rx recovers information modulated onto an RF carrier and provides the information to the receive (RX) processor 356. The TX processor 368 and the RX processor 356 implement layer 1 functionality associated with various signal processing functions. The RX processor 356 may perform spatial processing on the information to recover any spatial streams destined for the UE 350. If multiple spatial streams are destined for the UE 350, they may be combined by the RX processor 356 into a single OFDM symbol stream. The RX processor 356 then converts the OFDM symbol stream from the time-domain to the frequency domain using a Fast Fourier Transform (FFT). The frequency domain signal includes a separate OFDM symbol stream for each subcarrier of the OFDM signal. The symbols on each subcarrier, and the reference signal, are recovered and demodulated by determining the most likely signal constellation points transmitted by the base station 310. These soft decisions may be based on channel estimates computed by the channel estimator 358. The soft decisions are then decoded and deinterleaved to recover the data and control signals that were originally transmitted by the base station 310 on the physical channel. The data and control signals are then provided to the controller/processor 359, which implements layer 3 and layer 2 functionality.

The controller/processor 359 can be associated with a memory 360 that stores program codes and data. The memory 360 may be referred to as a computer-readable medium. In the UL, the controller/processor 359 provides demultiplexing between transport and logical channels, packet reassembly, deciphering, header decompression, and control signal processing to recover IP packets. The controller/processor 359 is also responsible for error detection using an ACK and/or NACK protocol to support HARQ operations.

Similar to the functionality described in connection with the DL transmission by the base station 310, the controller/processor 359 provides RRC layer functionality associated with system information (e.g., MIB, SIBs) acquisition, RRC connections, and measurement reporting; PDCP layer functionality associated with header compression / decompression, and security (ciphering, deciphering, integrity protection, integrity verification); RLC layer functionality associated with the transfer of upper layer PDUs, error correction through ARQ, concatenation, segmentation, and reassembly of RLC SDUs, re-segmentation of RLC data PDUs, and reordering of RLC data PDUs; and MAC layer functionality associated with mapping between logical channels and transport channels, multiplexing of MAC SDUs onto TBs, demultiplexing of MAC SDUs from TBs, scheduling information reporting, error correction through HARQ, priority handling, and logical channel prioritization.

Channel estimates derived by a channel estimator 358 from a reference signal or feedback transmitted by the base station 310 may be used by the TX processor 368 to select the appropriate coding and modulation schemes, and to facilitate spatial processing. The spatial streams generated by the TX processor 368 may be provided to different antenna 352 via separate transmitters 354Tx. Each transmitter 354Tx may modulate an RF carrier with a respective spatial stream for transmission.

The UL transmission is processed at the base station 310 in a manner similar to that described in connection with the receiver function at the UE 350. Each receiver 318Rx receives a signal through its respective antenna 320. Each receiver 318Rx recovers information modulated onto an RF carrier and provides the information to a RX processor 370.

The controller/processor 375 can be associated with a memory 376 that stores program codes and data. The memory 376 may be referred to as a computer-readable medium. In the UL, the controller/processor 375 provides demultiplexing between transport and logical channels, packet reassembly, deciphering, header decompression, control signal processing to recover IP packets. The controller/processor 375 is also responsible for error detection using an ACK and/or NACK protocol to support HARQ operations.

At least one of the TX processor 368, the RX processor 356, and the controller/processor 359 may be configured to perform aspects in connection with the exercise calculation component 198 of FIG. 1.

At least one of the TX processor 368, the RX processor 356, and the controller/processor 359 may be configured to perform aspects in connection with the control message reception component 199 of FIG. 1.

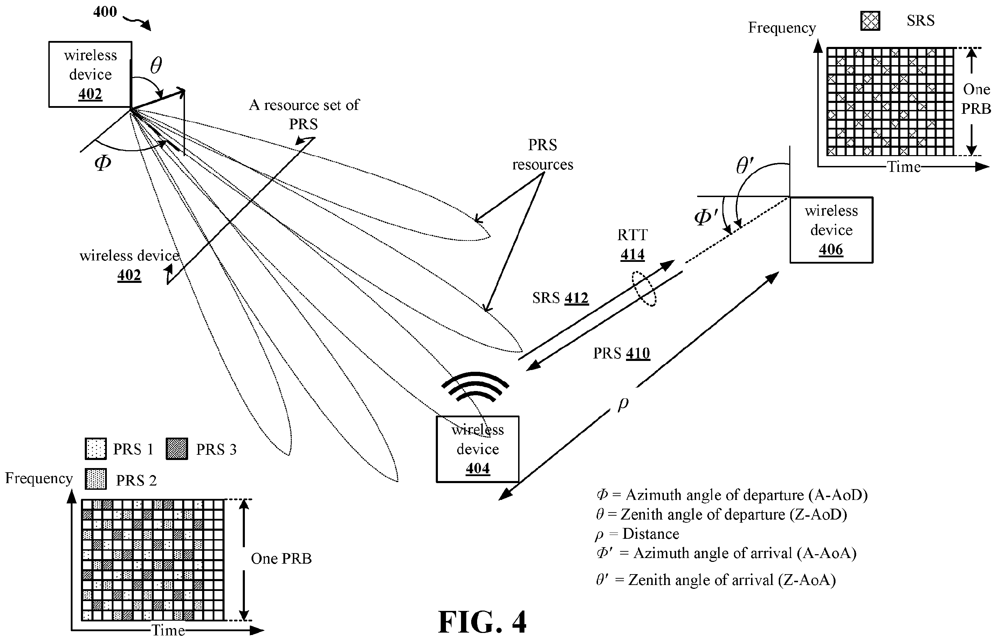

FIG. 4 is a diagram 400 illustrating an example of positioning based on reference signal measurements. The wireless device 402 may be a UE, a base station, or a positioning reference unit (PRU). The wireless device 404 may be a UE, a base station, or a PRU. The wireless device 406 may be a UE, a base station, or a PRU. The wireless device 402 may be referred to as a positioning target wireless device, whose location may be calculated based on measurements of one or more reference signals. The wireless device 404 and the wireless device 406 may be referred to as positioning neighbor wireless devices, whose locations may be known, which may be used to calculate the location of the wireless device 402. The wireless device 404 may transmit SRS 412 at time T_{SRS_TX} to the wireless device 406. The wireless device 404 may receive positioning reference signals (PRS) 410 at time T_{PRS_RX} from the wireless device 406. The SRS 412 may be an UL-SRS. The PRS 410 may be a DL-PRS. In some aspects, the wireless device 402 may be a TRP and the wireless device 406 may be a TRP, which may be both configured to transmit DL-PRS to the wireless device 404. The wireless device 404 may be a UE configured to transmit UL-SRS to the wireless device 402 and the wireless device 406.

The wireless device 406 may receive the SRS 412 at time T_{SRS_RX} from the wireless device 404 and transmit the PRS 410 at time T_{PRS_TX} to the wireless device 404. The wireless device 404 may receive the PRS 410 before transmitting the SRS 412. The wireless device 404 may transmit the SRS 412 before receiving the PRS 410. The wireless device 404 may transmit the SRS 412 in response to receiving the PRS 410. The wireless device 406 may transmit the PRS 410 in response to receiving the SRS 412. A positioning server (e.g., location server(s)168), the wireless device 404, or the wireless device 406 may determine the round-trip-time (RTT) 414 based on ∥T_{SRS_RX} - T_{PRS_TX}| - |T_{SRS_TX} - T_{PRS_RX}∥. Multi-RTT positioning may make use of the Rx-Tx time difference measurements (i.e., |T_{SRS_TX} - T_{PRS_RX}|) and PRS reference signal received power (RSRP) (PRS-RSRP) of PRS signals received from multiple wireless devices, such as the wireless device 402 and the wireless device 406, which are measured by the wireless device 404, and the measured Rx-Tx time difference measurements (i.e., |T_{SRS_RX} - T_{PRS_TX}|) and SRS-RSRP at multiple wireless devices, such as at the wireless device 402 and at the wireless device 406 of SRS transmitted from wireless device 404. The wireless device 404 may measure the Rx-Tx time difference measurements, and/or PRS-RSRP of the received signals, using assistance data received from the positioning server, the wireless device 402, and/or the wireless device 406. The wireless device 402 and the wireless device 406 may measure the Rx-Tx time difference measurements, and/or SRS-RSRP of the received signals, using assistance data received from the positioning server. The measurements may be used at the positioning server or the wireless device 404 to determine the RTT, which may be used to estimate the location of the wireless device 404. Other methods are possible for determining the RTT, such as for example using time-difference of arrival (TDOA) measurements, such as DL-TDOA and/or UL-TDOA measurements.

DL-AoD positioning may make use of the measured PRS-RSRP of signals transmitted from multiple wireless devices, such as the wireless device 402 and the wireless device 406, and received at the wireless device 404. The AoD positioning may also be referred to as DL-AoD positioning where the PRS are DL signals. The wireless device 404 may measure the PRS-RSRP of the received signals using assistance data received from the positioning server, and the resulting measurements may be used along with the azimuth angle of departure (A-AoD), the zenith angle of departure (Z-AoD), and other configuration information to locate the wireless device 404 in relation to the neighboring wireless devices that transmitted the PRS, such as the wireless device 402 and the wireless device 406.

DL-TDOA positioning may make use of the DL reference signal time difference (RSTD), and/or PRS-RSRP of signals received from multiple wireless devices, such as the wireless device 402 and the wireless device 406, at the wireless device 404. The wireless device 404 may measure the RSTD, and/or the PRS-RSRP, of the received PRS signals using assistance data received from the positioning server, and the resulting measurements may be used along with other configuration information to locate the wireless device 404 in relation to the neighboring wireless devices that transmitted the PRS, such as the wireless device 402 and the wireless device 406.

UL-TDOA positioning may make use of the UL relative time of arrival (RTOA), and/or SRS-RSRP, at multiple wireless devices, such as the wireless device 402 and the wireless device 406, of signals transmitted from the wireless device 404. The wireless devices, such as the wireless device 402 and the wireless device 406, may measure the RTOA, and/or the SRS-RSRP, of the received signals using assistance data received from the positioning server, and the resulting measurements may be used along with other configuration information to estimate the location of the wireless device 404.

UL-AoA positioning may make use of the measured azimuth angle of arrival (A-AoA) and zenith angle of arrival (Z-AoA) at multiple wireless devices, such as the wireless device 402 and the wireless device 406, of signals transmitted from the wireless device 404. The wireless device 402 and the wireless device 406 may measure the A-AoA and the Z-AoA of the received signals using assistance data received from the positioning server, and the resulting measurements may be used along with other configuration information to estimate the location of the wireless device 404.

Additional positioning methods may be used for estimating the location of the wireless device 404, such as for example, UL-AoD and/or DL-AoA at the wireless device 404. Note that data/measurements from various technologies may be combined in various ways to increase accuracy, to determine and/or to enhance certainty, to supplement/complement measurements, and/or to substitute/provide for missing information.

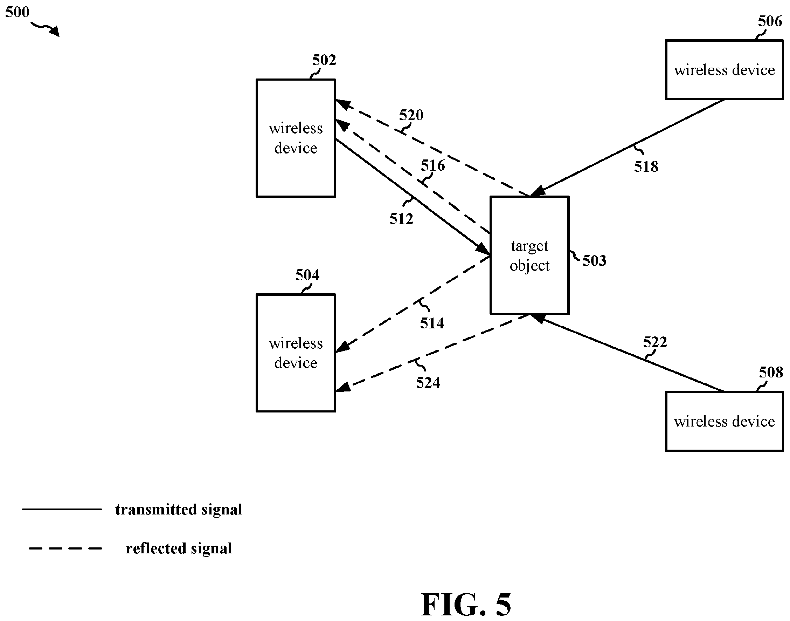

FIG. 5 is a diagram 500 illustrating an example of sensing based on measuring sensing signals transmitted by one or more sensing signals that reflect off of a target object 503. A wireless device that transmits a sensing signal that reflects off of a target object may be referred to as a transmitter node. A wireless device that receives a reflected sensing signal and measures the reflected sensing signal to perform sensing may be referred to as a receiver node. In one aspect, the wireless device 502 may perform monostatic sensing. The wireless device 502 may act as both a transmitter node and a receiver node. The wireless device 502 may transmit a set of sensing signals 512 at the target object 503, the target object 503 may reflect the set of sensing signals 512 as the reflected set of sensing signals 516 at the wireless device 502, and the wireless device 502 may measure the reflected set of sensing signals 516 from the target object 503. In another aspect, the wireless device 502 and the wireless device 504 may perform bistatic sensing. The wireless device 502 may act as a transmitter node and the wireless device 504 acts as a receiver node. The wireless device 502 may transmit a set of sensing signals 512 at the target object 503, the target object 503 may reflect the set of sensing signals 512 as the reflected set of sensing signals 514 at the wireless device 504, and the wireless device 504 may measure the reflected set of sensing signals 514 from the target object 503. In another aspect the wireless device 502 and the wireless device 506 may perform multi-static sensing. The wireless device 502 may act as both a transmitter node and a receiver node, for a first set of sensing signals, and the wireless device 506 acts as a transmitter node while the wireless device 502 acts as a receiver node for a second set of sensing signals. In addition to the wireless device 502 measuring the reflected set of sensing signals 516 from the target object 503 using monostatic sensing, the wireless device 506 may transmit a set of sensing signals 518 at the target object 503, the target object 503 may reflect the set of sensing signals 518 as the reflected set of sensing signals 520 at the wireless device 502, and the wireless device 502 may measure the reflected set of sensing signals 520 from the target object 503. In another aspect the wireless device 502, the wireless device 504, and the wireless device 508 may perform multi-static sensing. The wireless device 502 may act as a transmitter node and the wireless device 504 acts as a receiver node for a first set of sensing signals, and the wireless device 508 acts as a transmitter node and the wireless device 504 acts as a receiver node for a second set of sensing signals. In addition to the wireless device 504 measuring the reflected set of sensing signals 514 from the target object 503 using bistatic sensing, the wireless device 508 may transmit a set of sensing signals 522 at the target object 503, the target object 503 may reflect the set of sensing signals 522 as the reflected set of sensing signals 524 at the wireless device 504, and the wireless device 504 may measure the reflected set of sensing signals 524 from the target object 503. Each wireless device may be any wireless device configured to transmit or receive wireless signals, such as UEs, network nodes, TRPs, or base stations. For example, the wireless device 502 may be a network node configured to transmit the set of sensing signals 512 at the target object 503 and measure the reflected set of sensing signals 516 from the target object 503. In another example, the wireless device 502 may be a network node configured to transmit the set of sensing signals 512 at the target object 503, and the wireless device 504 may be a UE configured to measure the reflected set of sensing signals 514 from the target object 503.

The wireless device 502 may conduct one or more sensing measurements on the reflected set of sensing signals 516 and/or the reflected set of sensing signals 520. In one aspect, the wireless device 502 may calculate a distance or a range between the wireless device 502 and the target object 503 based on a round trip time (RTT) between when the wireless device 502 transmits the set of sensing signals 512 and when the wireless device 502 receives the reflected set of sensing signals 516. In one aspect, the wireless device 502 may calculate a distance or a range that the set of sensing signals 518 and the reflected set of sensing signals 520 travels based on a time between when the wireless device 506 transmits the set of sensing signals 518 and when the wireless device 502 receives the reflected set of sensing signals 520. In one aspect, the wireless device 502 may calculate a location of the target object 503 based on a plurality or range or distance measurements, for example via triangulation using known positions of the wireless devices 502 and 506 and the calculated range or distance measurements. In one aspect, the wireless device 502 may calculate a velocity of the target object 503 based on a first calculated location of the target object 503 based on the reflected set of sensing signals 516 and/or the reflected set of sensing signals 520 measured at a first time, and a second calculated location of the target object 503 based on the reflected set of sensing signals 516 and/or the reflected set of sensing signals 520 measured at a second time. In one aspect, the wireless device 502 may calculate an AoA of the reflected set of sensing signals 516 and/or an AoD of the set of sensing signals 512 based on a plurality of ports that transmitted the set of sensing signals 512 and a plurality of ports that received the reflected set of sensing signals 516. In one aspect, the wireless device 502 may calculate an AoA of the reflected set of sensing signals 520 and/or an AoD of the set of sensing signals 518 based on a plurality of ports that transmitted the set of sensing signals 518 and a plurality of ports that received the reflected set of sensing signals 520.

Similarly, the wireless device 504 may conduct one or more sensing measurements on the reflected set of sensing signals 514 and/or the reflected set of sensing signals 524. In one aspect, the wireless device 504 may calculate a distance or a range that the set of sensing signals 512 and the reflected set of sensing signals 514 travels based on a time between when the wireless device 502 transmits the set of sensing signals 512 and when the wireless device 504 receives the reflected set of sensing signals 514. In one aspect, the wireless device 504 may calculate a distance or a range that the set of sensing signals 522 and the reflected set of sensing signals 524 travels based on a time between when the wireless device 508 transmits the set of sensing signals 522 and when the wireless device 504 receives the reflected set of sensing signals 524. In one aspect, the wireless device 504 may calculate a location of the target object 503 based on a plurality or range or distance measurements, for example via triangulation using the known positions of wireless devices 502, 504, and 508, and the calculated range or distance measurements. In one aspect, the wireless device 504 may calculate a velocity of the target object 503 based on a first calculated location of the target object 503 based on the reflected set of sensing signals 514 and/or the reflected set of sensing signals 524 measured at a first time, and a second calculated location of the target object 503 based on the reflected set of sensing signals 514 and/or the reflected set of sensing signals 524 measured at a second time. In one aspect, the wireless device 504 may calculate an AoA of the reflected set of sensing signals 514 and/or an AoD of the set of sensing signals 512 based on a plurality of ports that transmitted the set of sensing signals 512 and a plurality of ports that received the reflected set of sensing signals 514. In one aspect, the wireless device 504 may calculate an AoA of the reflected set of sensing signals 524 and/or an AoD of the set of sensing signals 522 based on a plurality of ports that transmitted the set of sensing signals 522 and a plurality of ports that received the reflected set of sensing signals 524.

While a wireless device may sense parameters of the target object 503 by measuring a reflected set of sensing signals originating from a transmitter node, such a wireless device may improve its sensing by measuring two or more reflected sets of sensing signals originating from two or more transmitter nodes. For example, the wireless device 502 may improve its sensing by measuring the reflected set of sensing signals 516 originating from the wireless device 502 as the set of sensing signals 512 in addition to measuring the reflected set of sensing signals 520 originating from the wireless device 506 as the set of sensing signals 518. In another example, the wireless device 504 may improve its sensing by measuring the reflected set of sensing signals 514 originating from the wireless device 502 as the set of sensing signals 512 in addition to measuring the reflected set of sensing signals 524 originating from the wireless device 508 as the set of sensing signals 522.

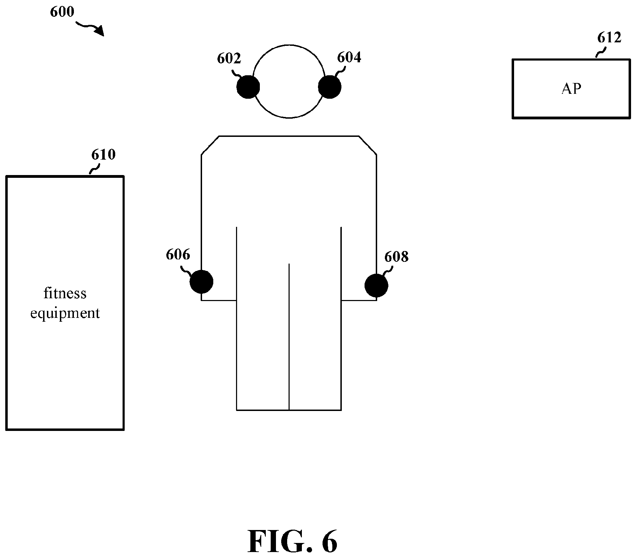

FIG. 6 is a diagram illustrating a user 600 wearing a set of UEs (i.e., UE 602, UE 604, UE 606, UE 608) that may be used to calculate an exercise state of the user 600. The user 600 may wear the set of UEs in any suitable manner. For example, the UE 602 and the UE 604 may be earbuds that the UE wears in or about the user's left and right ears. In some aspects, the UE 602 and the UE 604 may include wireless earbuds that the user 600 may use while working out. The UE 606 may be a smartphone of the user held in the user's hand or strapped to a wrist of the user. The UE 608 may be a smart watch worn about the wrist of the user or may be a smart ring worn on a finger of the user. A UE may be an enhanced ranging device (ERDEV) that calculates a distance and/or angle of the ERDEV with respect to other ERDEVs. An ERDEV may be a controller that controls the ranging and defines the ranging parameters via a ranging control message (RCM). An ERDEV may be a controllee that utilizes ranging parameters received in a control message, such as an RCM, from the controller. An ERDEV may be an initiator that initiates a ranging exchange by transmitting an initiation message, such as a ranging initiation message (RIM) to one or more other ERDEVs. An initiator may transmit the initiation message in response to receiving a control message, such as an RCM. An initiator may be a controller or a controllee. An ERDEV may be a responder that transmits a response message, such as a ranging response message (RRM) to one or more ERDEVs. The responder may transmit the response message in response to receiving an initiation message. A responder may be a controller or a controllee.

A UE may have a set of sensors that allow the UE to collect measurements to train an exercise detection model. Such sensors may include, for example, an antenna to measure positioning signals, an antenna to measure sensing signals, a motion sensor (e.g., IMU, gyroscope, accelerometer) to measure movements, a light sensor to measure light, a GNSS sensor to measure a location, an altimeter to measure altitude, a barometer to measure pressure, a sound sensor to measure sounds, and/or a magnetometer to measure magnetic waves. The UE may collect such measurements and feed them to a training module for training an exercise detection model, or may feed them to a trained exercise detection model to detect an exercise state of a user. In some aspects, such computations may be made locally by the UE (e.g., a smart watch or a smart ring). In some aspects, such computations may be made locally by a more powerful UE that receives sensor information from a set of UEs (e.g., a powerful smartphone within a zone of the set of UEs, a remote server that receives sensor information, a private server that receives sensor information). In some aspects, additional measurements may be made by other UEs that communicate with a device with the exercise detection model, such as fitness equipment, or an AP (e.g., a Wi-Fi, UWB, or BLE AP). Any of the devices that perform measurements may aggregate sensor information from the UEs to calculate an exercise state of the user 600 based on sensor information.

One of the UEs may transmit positioning signals to the other UEs to calculate relative ranges/angles between the set of UEs. For example, the UE 602 may transmit a positioning signal to the UE 604, the UE 606, and the UE 608. In response to receiving the positioning signals, the UE 604, the UE 606, and the UE 608 may respond with a positioning signal back to the UE 602. The UE 602 may then calculate relative ranges between the UE 602 and each of the UE 604, the UE 606, and the UE 608, for example by calculating RTT. In other aspects, the UE 602 may broadcast a positioning signal, and the other UEs, UE 604, UE 606, and UE 608, may record ToAs of the broadcast signal to calculate relative ranges.

One of the UEs may transmit sensing signals to the other UEs, or to parts of the body of the user 600, to calculate relative ranges/angles between the set of UEs, or between the transmitting UE and parts of the body of the user 600. For example, the UE 608 may transmit a sensing signal that reflects off of the UE 602, the UE 604, the UE 606, and/or parts of the body of the user 600. Any of the UEs may receive the reflected sensing signals to calculate the relative ranges/angles of the target object that reflected the sensing signal and the transmitting and/or receiving device. For example, the UE 602 may receive a reflected sensing signal to calculate a distance between the target object and the UE 608 that transmitted the sensing signal, to calculate a distance between the target object and the UE 602 that receive the reflected sensing signal, an AoD of the sensing signal transmitted from the UE 608, or an AoA of the reflected sensing signal received by the UE 602.

One of the UEs may aggregate the measurements, or calculated results of the measurements (e.g., RTT, ToA, AoA, calculated range, calculated angle) to determine an exercise state of the user. For example, the aggregated measurements may indicate that the user 600 is walking, is running, or is jumping. In some aspects, an idealized movement state may be saved by the analyzing device. The idealized movement state may also be referred to as a reference movement. The UE may determine the exercise state by determining that the analyzed movement is within a threshold of the reference movement by a threshold amount. The thresholds may be a function of the relative ranges and angles between a pair of devices. The thresholds may be relative to whether the movements follow a periodic pattern for a threshold minimum period of time. In response to determining the exercise state, the analyzing device may compare the exercise state of the idealized movement state to suggest how the user 600 may improve their form (e.g., lift their leg higher when jumping, step forward more while performing a lunge).

In some aspects, a pair of UEs, for example the UE 602 and the UE 608, may initiate an ERDEV UWB session between the two devices to perform positioning relative to the two devices. A UWB session may include consecutive ranging blocks, where each block may include a set of ranging rounds. Each raging round may include a set of ranging slots. A ranging round may include a ranging control phase of one ranging slot, a ranging phase of a plurality of ranging slots (e.g., transmission and measurement of positioning signals), and a measurement report phase of a plurality of ranging slots (e.g., reporting of positioning signal measurements). In other words, a ranging round may include a single slot for the control phase, followed by the ranging phase, followed by the measurement report phase. Within a ranging block, a responder device may transmit a message within a single round. A controller may statically configure the round index, or may be selected by a UE in accordance with a hopping pattern. The UEs may use slots within a chosen round sequentially to calculate ranging (i.e., distance between the UEs) or TDoA (if several UEs receive and measure the positioning signals from a single transmitting UE).

An exercise detection model utilizing inputs measured by the UE 602, the UE 604, the UE 606, and the UE 608 may calculate a relative range between pairs of the UEs. The relative range may be calculated based on ToA and/or TDoA estimates. An exercise detection model utilizing inputs measured by the UE 602, the UE 604, the UE 606, and the UE 608 may calculate a relative angle between pairs of the UEs. The relative angle may be calculated based on AoA estimates. An exercise detection model utilizing inputs measured by the UE 602, the UE 604, the UE 606, and the UE 608 may calculate target object locations of parts of the user 600 that reflect sensing signals. The sensing measurements may be calculated based on CIRs. One or more of the UE 602, the UE 604, the UE 606, and/or the UE 608 may have one or more other sensors that may be provided to an exercise detection model, such as an IMU or a heartbeat monitor. Based on the inputs, the exercise detection model may determine whether the user 600 has started to exercise and the type of exercise. Based on the inputs, the exercise detection model may determine whether the user 600 is resting. Based on the inputs, the exercise detection model may determine whether the user 600 is performing the exercise correctly (e.g., the user's form can be improved).

In some aspects, a set of inputs may be collected over a period of time defined by a controlling device, such as a smartphone UE of the user 600. The time period may be a time period within which measurement data is buffered at a UE that has the exercise detection block. The exercise detection block may calculate mobility information of the user 600 based on the set of inputs collected over the period of time. For example, the relative range between UE 602 and UE 606 and between UE 604 and UE 608 may follow a periodic pattern over time, which may be mapped to a type of motion or exercise activity.

In some aspects, a UE may transmit a set of measurements to an offline device, such as an over the top (OTT) server, or a remote server. For example, a smartphone may collect measurements from the UE 602, the UE 604, the UE 606, and the UE 608 (or one of the UE 602, the UE 604, the UE 606, or the UE 608 may be a smartphone) and may transmit the measurements to a remote server, such as a connected intelligent edge (CIE) or OTT server, which may train an exercise detection model. Such a server may crowdsource measurement data from a plurality of UEs to develop the exercise detection model. Similarly, a UE may download a trained exercise detection model from such a remote device to calculate exercise activity of the user 600. In some aspects, the exercise detection model may be trained based on attributes of the UEs. For example, an exercise detection model may be associated with a set of devices belonging to a certain make/model, or to a set of devices with a certain set of makes/models. In some aspects, an exercise detection model may be trained based on attributes of the user 600. For example, an exercise detection model may be associated with users having a certain height, width, and weight within threshold values. An exercise detection model may be trained with a large dataset of inputs from a plurality of crowdsourced devices to help train and deploy highly accurate exercise detection models. In some aspects, a UE may periodically download an exercise detection model from an offline device, such as a remote server or a CIE, to calculate an exercise state of the user 600.

In some aspects, a piece of fitness equipment, such as the equipment 610, may communicate with the UE 602, the UE 604, the UE 606, and/or the UE 608 to determine an exercise state of the user 600. The equipment 610 may be any suitable exercise equipment, such as a treadmill, an elliptical machine, a bicycle, or an interactive display. In some aspects, the equipment 610 may be a UE or a network node. The equipment 610 may transmit positioning signals and/or sensing signals to be measured by the UE 602, the UE 604, the UE 606, and/or the UE 608. The equipment 610 may measure positioning signals and/or reflected sensing signals transmitted by the UE 602, the UE 604, the UE 606, and/or the UE 608. In some aspects, the equipment 610 may act as a UWB initiator that initiates a UWB session with the UE 602, the UE 604, the UE 606, and/or the UE 608. In some aspects, the equipment 610 may aggregate measured sensing data from the UE 602, the UE 604, the UE 606, and/or the UE 608. In some aspects, the equipment 610 may have sensors that collect data from the user 600, such as antenna for conducting monostatic sensing on the user 600, a camera that captures movements of the user 600, or a heartbeat sensor that captures heartbeats of the user 600. In some aspects, the equipment 610 may have an exercise detection model stored on its memory. After collecting and processing the measurements using the exercise detection model, the equipment 610 may display exercise state statistics on a display to the user 600. In some aspects, the equipment 610 may provide cues or prompt the user 600 to correct their form based on the detected exercise state. For example, if an exercise state of the user 600 diverges from a reference movement by more than a threshold amount (e.g., the user 600 lifts an arm by more than 20% of a reference movement for a run), the equipment 610 may prompt the user to perform their exercise closer to the reference movement (e.g., prompt the user to lift their arm by a lesser amount while running). The equipment 610 may store a reference movement based on the type of equipment, such as reference movements for jogging for an exercise bike or reference movements for biking for a stationary bicycle. In some aspects, the equipment 610 may have a first exercise detection model and a UE worn by the user 600, such as the UE 606, may have a second exercise detection model different from the first exercise detection model. The equipment 610 may have an exercise detection model that detects an exercise state of the user 600 based on measurements collected by the UE 602, the UE 604, the UE 606, the UE 608, and the equipment 610, while the UE 606 may have an exercise detection model that detects an exercise state of the user 600 based on measurements collected by the UE 602, the UE 604, the UE 606, and the UE 608. In some aspects, the first exercise detection model may be specific to a certain type/made/model of fitness equipment associated with the equipment 610 and/or the UEs worn by the user 600. In some aspects, the first exercise detection model may be trained on the equipment 610.

In some aspects, an access point (AP), such as the AP 612, may be used to transmit positioning signals and/or sensing signals at the user 600, or to measure positioning signals and/or sensing signals at the user 600. In some aspects, the equipment 610 may include an AP. The AP 612 may support RF signals different than the UE 602, the UE 604, the UE 606, and/or the UE 608. For example, the UE 602, the UE 604, the UE 606, and/or the UE 608 may be configured to transmit UWB signals, and the AP 612 may be configured to transmit Wi-Fi signals and/or BLE signals. In some aspects, the AP 612 may perform monostatic sensing on the user 600. In some aspects, a set of APs may work cooperatively about the user 600 to perform measurements on the user 600 along with the UE 602, the UE 604, the UE 606, and/or the UE 608. For example, the user 600 may exercise within a studio having a plurality of APs. In some aspects, the AP 612 may have a first exercise detection model and a UE worn by the user 600, such as the UE 606, may have a second exercise detection model different from the first exercise detection model. The AP 612 may have an exercise detection model that detects an exercise state of the user 600 based on measurements collected by the UE 602, the UE 604, the UE 606, the UE 608, and the AP 612, while the UE 606 may have an exercise detection model that detects an exercise state of the user 600 based on measurements collected by the UE 602, the UE 604, the UE 606, and the UE 608. In some aspects, the first exercise detection model may be specific to a certain type/made/model of fitness equipment associated with the AP 612 and/or the UEs worn by the user 600. In some aspects, the first exercise detection model may be trained on the AP 612. In some aspects, the inputs to train the exercise detection model at the AP 612 may include locations of one or more APs about the user 600 that transmit and/or measure positioning signals and/or sensing signals.

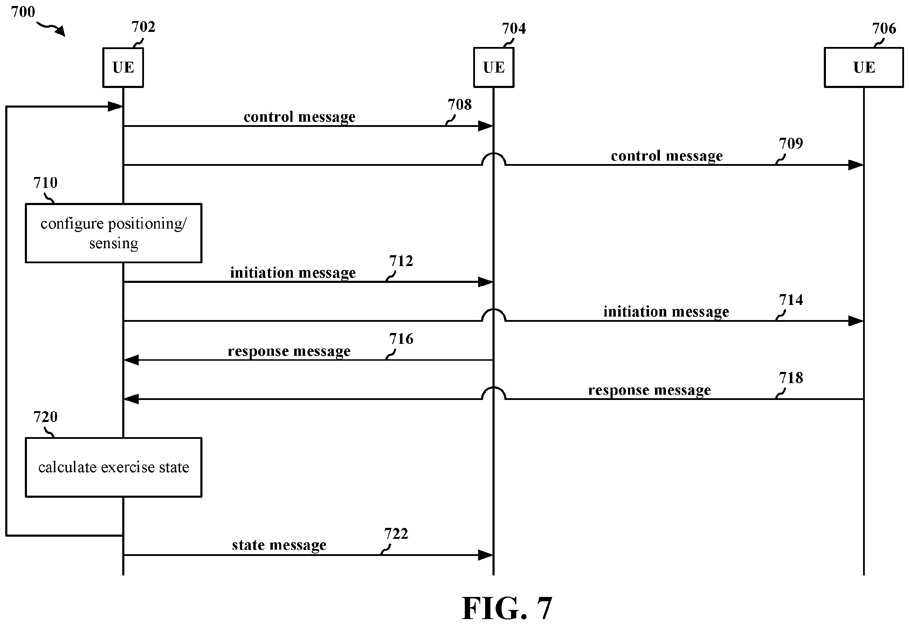

FIG. 7 is a connection flow diagram 700 between a set of UEs (e.g., UE 702, UE 704, UE 706) configured to calculate an exercise state of a user associated with the set of UEs. The UE 702 may be an initiator that has an exercise detection block that may be used to calculate an exercise state of the user. The UE 702 may be, for example, a smart watch or a smart ring. The UE 702 may transmit a control message 708 to the UE 704. The UE 702 may transmit a control message 709 to the UE 706. The control message 708 and/or the control message 709 may be a ranging control message (RCM). The control message may prime the UE 704 and/or the UE 706 to respond to a positioning signal transmitted to the UE. At 710, the UE 702 may configure a positioning occasion, and/or a sensing occasion. The UE 702 may transmit an initiation message 712 to the UE 704. The initiation message 712 may be a ranging initiation message (RIM). The initiation message 712 may be, for example, a positioning signal (e.g., an SRS, a PRS, a CSI-RS) or may be a sensing signal. In response to receiving the initiation message 712, the UE 704 may transmit a response message 716 at the UE 702. The response message 716 may be a ranging response message (RRM). The UE 702 may transmit an initiation message 714 to the UE 706. The initiation message 714 may be a RIM. The initiation message 714 may be, for example, a positioning signal or may be a sensing signal. In response to receiving the initiation message 714, the UE 706 may transmit a response message 718 at the UE 702. The response message 718 may be an RRM.

In some aspects, the initiation message 712 may be a positioning signal. The UE 704 may measure the initiation message 712. The response message 716 may include a measurement of the positioning signal. In some aspects, the initiation message 712 may be a sensing signal. The UE 704 may measure a reflection of the sensing signal off of a target object. The response message 716 may include a measurement of the sensing signal. In some aspects, the initiation message 712 may be a configuration of positioning signal. The response message 716 may be a positioning signal. The UE 702 may measure the received positioning signal. In some aspects, the initiation message 712 may be a configuration of a sensing signal. The response message 716 may be a sensing signal. The UE 702 may measure a received reflection of the sensing signal. In some aspects, the response message 716 may include a positioning signal or a sensing signal having a payload of sensor data, such as a measurement of the initiation message 712 or sensor data from a sensor of the UE 704 (e.g., IMU data). The UE 702 may measure the positioning signal or a reflection of the sensing signal, and may also decode the payload to read the sensor data. In some aspects, the UE 704 may be configured to transmit the sensor data based on a time window. For example, an out of band (OOB) setup for the UE 704 may configure the UE 704 to transmit sensor data from an IMU periodically over a five-second time window.

In some aspects, the initiation message 714 may be a positioning signal. The UE 706 may measure the initiation message 714. The response message 718 may include a measurement of the positioning signal. In some aspects, the initiation message 714 may be a sensing signal. The UE 706 may measure a reflection of the sensing signal off of a target object. The response message 718 may include a measurement of the sensing signal. In some aspects, the initiation message 714 may be a configuration of positioning signal. The response message 718 may be a positioning signal. The UE 702 may measure the received positioning signal. In some aspects, the initiation message 714 may be a configuration of a sensing signal. The response message 718 may be a sensing signal. The UE 702 may measure a received reflection of the sensing signal. In some aspects, the response message 718 may include a positioning signal or a sensing signal having a payload of sensor data, such as a measurement of the initiation message 714 or sensor data from a sensor of the UE 706 (e.g., IMU data). The UE 702 may measure the positioning signal or a reflection of the sensing signal, and may also decode the payload to read the sensor data. In some aspects, the UE 706 may be configured to transmit the sensor data based on a time window. For example, an out of band (OOB) setup for the UE 706 may configure the UE 706 to transmit sensor data from an IMU periodically over a five-second time window.

At 720, the UE 702 may calculate an exercise state of the user based on the response message 716 and/or the response message 718. While FIG. 7 shows the UE 702 communicating with two UEs, the UE 704 and the UE 706, a UE may communicate with any number of UEs. In some aspects, the response message 716 and the response message 718 may relate with one another. For example, the initiation message 712 and the initiation message 714 may be an SRS, and the response message 716 and the response message 718 may indicate relative times of arrival of the SRS. The UE 702 may then calculate a difference between the time of arrival of the SRS at each of the UE 704 and the UE 706.

In some aspects, in response to the UE 702 calculating the exercise state of the user, the UE may transmit an updated control message to the UE 704 and/or the UE 706. For example, the UE 702 may update a periodicity of measurements in response to detecting that the user is increasing a speed of movement (e.g., running at 10 mph as opposed to jogging at 5 mph). In response to a measured value meeting or exceeding a threshold value, the UE 702 may indicate more frequency UWB measurement exchanges (e.g., a periodic measurement every two seconds instead of every five seconds). The control message 708 and/or the control message 709 may be a ranging control update message (RCUM) indicating a change from the previous control message.

In some aspects, the UE 702 may display and/or store the calculated exercise state on the UE 702. For example, the UE 702 may display the calculated exercise state on a screen of the UE 702. In some aspects, the UE 702 may transmit a state message 722 to the UE 704. The UE 704 may receive the state message 722. The UE 704 may then display and/or store the calculated exercise state on the UE 704.

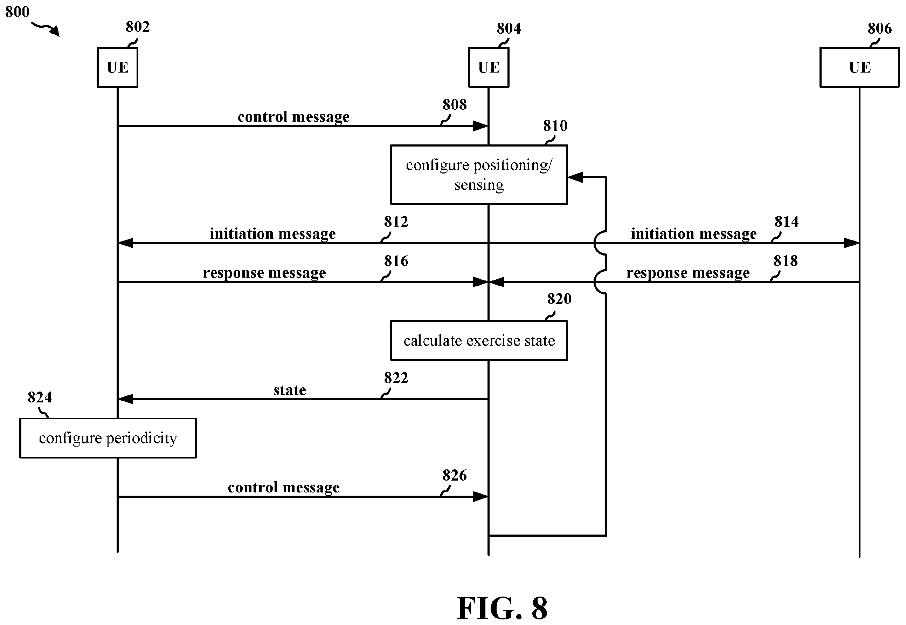

FIG. 8 is a connection flow diagram 800 between a set of UEs (e.g., UE 802, UE 804, UE 806) configured to calculate an exercise state of a user associated with the set of UEs. The UE 802 may transmit a control message 808 to the UE 804. The control message 808 may be a ranging control message (RCM). The control message 808 may trigger the UE 804 to configure a positioning occasion and/or a sensing occasion. At 810, the UE 804 may configure a positioning occasion, and/or a sensing occasion in response to the control message 808. The UE 804 may transmit an initiation message 812 to the UE 802. The initiation message 812 may be a RIM. The initiation message 812 may be, for example, a positioning signal or may be a sensing signal. In response to receiving the initiation message 812, the UE 802 may transmit a response message 816 at the UE 804. The response message 816 may be an RRM. The UE 804 may transmit an initiation message 814 to the UE 806. The initiation message 814 may be a RIM. The initiation message 814 may be, for example, a positioning signal or may be a sensing signal. In response to receiving the initiation message 814, the UE 806 may transmit a response message 818 at the UE 804.

At 820, the UE 804 may calculate an exercise state of the user based on the response message 816 and/or the response message 818. The UE 804 may transmit the calculated exercise state 822 to the UE 802 based on the control message 808. At 824, the UE 802 may configure a periodicity of the positioning occasion and/or sensing occasion. For example, the UE 802 may determine that the user is increasing a rate of movement. The UE 802 may transmit a control message 826 to the UE 804 indicating to the UE 804 to decrease the periodicity of its positioning/sensing occasions. The control message 826 may be a ranging control update message (RCUM). The UE 804 may then reconfigure its positioning/sensing occasion(s) at 810 in response to the control message 826. The UE 802 may display and/or store the calculated exercise state on the UE 802. For example, the UE 802 may display the calculated exercise state on a screen of the UE 802.

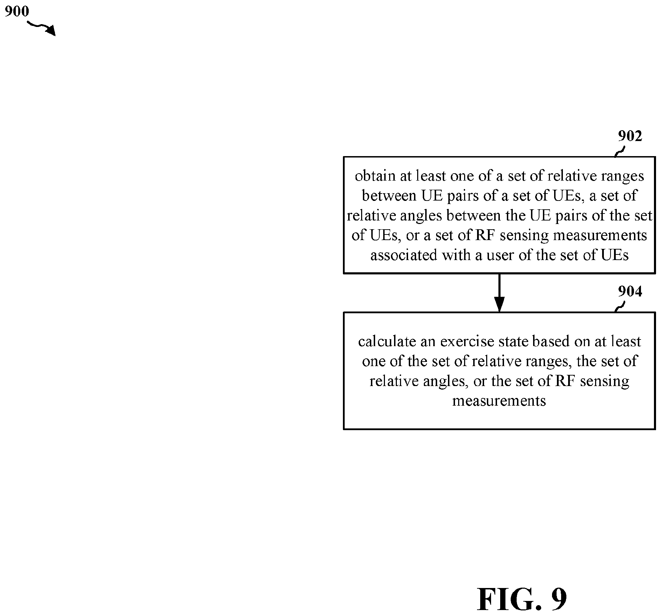

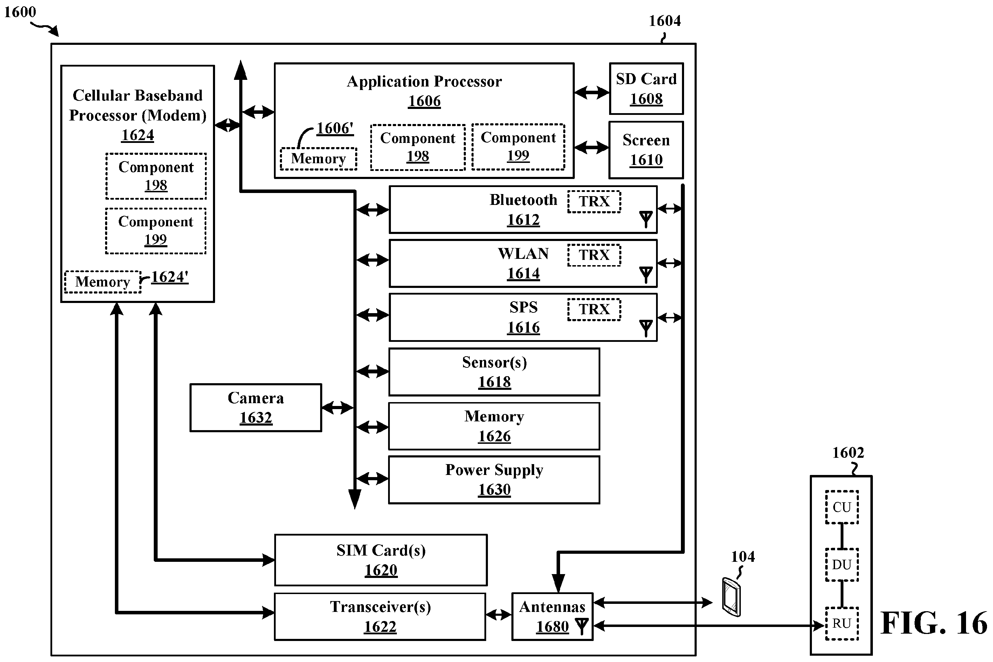

FIG. 9 is a flowchart 900 of a method of wireless communication. The method may be performed by a UE (e.g., the UE 104, the UE 350, the UE 602, the UE 604, the UE 606, the UE 608, the UE 702, the UE 704, the UE 706; the equipment 610; the AP 612; the wireless device 402, the wireless device 404, the wireless device 406, the wireless device 502, the wireless device 504, the wireless device 506, the wireless device 508; the apparatus 1604). At 902, the UE may obtain at least one of a set of relative ranges between UE pairs of a set of UEs, a set of relative angles between the UE pairs of the set of UEs, or a set of RF sensing measurements associated with a user of the set of UEs. For example, 902 may be performed by the UE 602 in FIG. 6, which may obtain at least one of a set of relative ranges between UE pairs (e.g., between the UE 602 and the UE 604) of the set of UEs (e.g., the UE 602, the UE 604, the UE 606, and the UE 608), a set of relative angles between the UE pairs of the set of UEs, or a set of RF sensing measurements associated with the user 600 of the set of UEs. Moreover, 902 may be performed by the component 198 in FIG. 16.

At 904, the UE may calculate an exercise state based on at least one of the set of relative ranges, the set of relative angles, or the set of RF sensing measurements. For example, 904 may be performed by the UE 602 in FIG. 6, which may calculate an exercise state of the user 600 of the UE 602, the UE 604, the UE 606, and the UE 608 based on at least one of the set of relative ranges, the set of relative angles, or the set of RF sensing measurements. Moreover, 904 may be performed by the component 198 in FIG. 16.

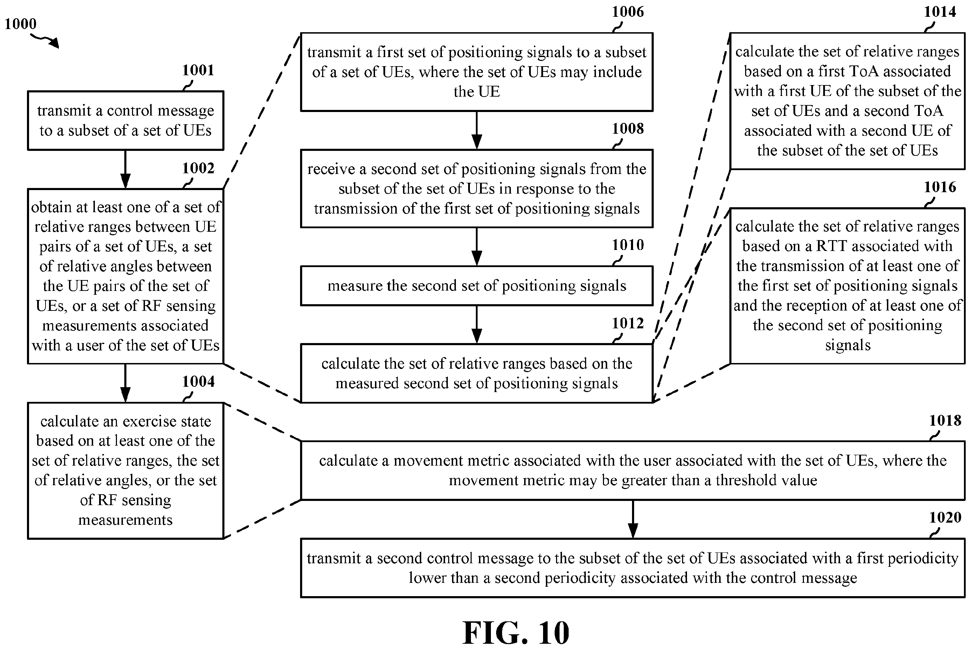

FIG. 10 is a flowchart 1000 of a method of wireless communication. The method may be performed by a UE (e.g., the UE 104, the UE 350, the UE 602, the UE 604, the UE 606, the UE 608, the UE 702, the UE 704, the UE 706; the equipment 610; the AP 612; the wireless device 402, the wireless device 404, the wireless device 406, the wireless device 502, the wireless device 504, the wireless device 506, the wireless device 508; the apparatus 1604). At 1001, the UE may transmit a control message to a subset of a set of UEs. For example, 1001 may be performed by the UE 602 in FIG. 6, which may transmit a control message to a subset of the set of UEs. The set of UEs may include the UE 604, the UE 606, the UE 608, the equipment 610, and/or the AP 612. Moreover, 1001 may be performed by the component 198 in FIG. 16.

At 1002, the UE may obtain at least one of a set of relative ranges between UE pairs of a set of UEs, a set of relative angles between the UE pairs of the set of UEs, or a set of RF sensing measurements associated with a user of the set of UEs. For example, 1002 may be performed by the UE 602 in FIG. 6, which may obtain at least one of a set of relative ranges between UE pairs (e.g., between the UE 602 and the UE 604) of the set of UEs (e.g., the UE 602, the UE 604, the UE 606, and the UE 608), a set of relative angles between the UE pairs of the set of UEs, or a set of RF sensing measurements associated with the user 600 of the set of UEs. Moreover, 1002 may be performed by the component 198 in FIG. 16.

At 1004, the UE may calculate an exercise state based on at least one of the set of relative ranges, the set of relative angles, or the set of RF sensing measurements. For example, 1004 may be performed by the UE 602 in FIG. 6, which may calculate an exercise state of the user 600 of the UE 602, the UE 604, the UE 606, and the UE 608 based on at least one of the set of relative ranges, the set of relative angles, or the set of RF sensing measurements. Moreover, 1004 may be performed by the component 198 in FIG. 16.

At 1006, the UE may obtain the set of relative ranges between UE pairs of the set of UEs by transmitting a first set of positioning signals to a subset of a set of UEs, where the set of UEs may include the UE. For example, 1006 may be performed by the UE 602 in FIG. 6, which may transmit a first set of positioning signals to the UE 604, the UE 606, and the UE 608 of the set of UEs that include the UE 602, the UE 606, the UE 608, the equipment 610, and the AP 612. The set of UEs that include the UE 602, the UE 606, the UE 608, the equipment 610, and the AP 612 may include the UE 602. Moreover, 1006 may be performed by the component 198 in FIG. 16.

At 1008, the UE may obtain the set of relative ranges between UE pairs of the set of UEs by receiving a second set of positioning signals from the subset of the set of UEs in response to the transmission of the first set of positioning signals. For example, 1008 may be performed by the UE 602 in FIG. 6, which may receive a second set of positioning signals from the UE 604, the UE 606, and the UE 608 of the set of UEs that include the UE 602, the UE 606, the UE 608, the equipment 610, and the AP 612. The second set of positioning signals received from the UE 604, the UE 606, and the UE 608 may be in response to the transmission of the first set of positioning signals. Moreover, 1008 may be performed by the component 198 in FIG. 16.

At 1010, the UE may obtain the set of relative ranges between UE pairs of the set of UEs by measuring the second set of positioning signals. For example, 1010 may be performed by the UE 602 in FIG. 6, which may measure the second set of positioning signals. Moreover, 1010 may be performed by the component 198 in FIG. 16.

At 1012, the UE may obtain the set of relative ranges between UE pairs of the set of UEs by calculating the set of relative ranges based on the measured second set of positioning signals. For example, 1012 may be performed by the UE 602 in FIG. 6, which may calculate the set of relative ranges between the UE 602 and the UE 604, the UE 606, and the UE 608 based on the measured second set of positioning signals. Moreover, 1012 may be performed by the component 198 in FIG. 16.

At 1014, the UE may calculate the set of relative ranges based on the measured second set of positioning signals by calculating the set of relative ranges based on a first ToA associated with a first UE of the subset of the set of UEs and a second ToA associated with a second UE of the subset of the set of UEs. For example, 1014 may be performed by the UE 602 in FIG. 6, which may calculate the set of relative ranges based on a first ToA associated with the UE 604 of the subset of the set of UEs and a second ToA associated with the UE 606 of the subset of the set of UEs. Moreover, 1014 may be performed by the component 198 in FIG. 16.

At 1016, the UE may calculate the set of relative ranges based on the measured second set of positioning signals by calculating the set of relative ranges based on a RTT associated with the transmission of at least one of the first set of positioning signals and the reception of at least one of the second set of positioning signals. For example, 1016 may be performed by the UE 602 in FIG. 6, which may calculate the set of relative ranges based on a RTT associated with the transmission of at least one of the first set of positioning signals and the reception of at least one of the second set of positioning signals. Moreover, 1016 may be performed by the component 198 in FIG. 16.

At 1018, the UE may calculate the exercise state by calculating a movement metric associated with the user associated with the set of UEs, where the movement metric may be greater than a threshold value. For example, 1018 may be performed by the UE 602 in FIG. 6, which may calculate a movement metric associated with the user 600 associated with the set of UEs. The movement metric may be greater or equal to a threshold value. The UE 602 may detect that the user 600 is moving at a greater speed in response to the movement metric being greater or equal to the threshold value. Moreover, 1018 may be performed by the component 198 in FIG. 16.

At 1020, the UE may transmit a second control message to the subset of the set of UEs associated with a first periodicity lower than a second periodicity associated with the control message. For example, 1020 may be performed by the UE 602 in FIG. 6, which may transmit a second control message to the UE 604, the UE 606, and the UE 608 of the set of UEs. The second control message may be associated with a first periodicity lower than a second periodicity associated with the control message, increasing the frequency of periodic updates from the UE 604, the UE 606, and the UE 608 of the set of UEs based on detecting that the user 600 is moving at a greater speed. This may allow the UE 602 to detect motions from the user 600 with a greater degree of granularity. Moreover, 1020 may be performed by the component 198 in FIG. 16.

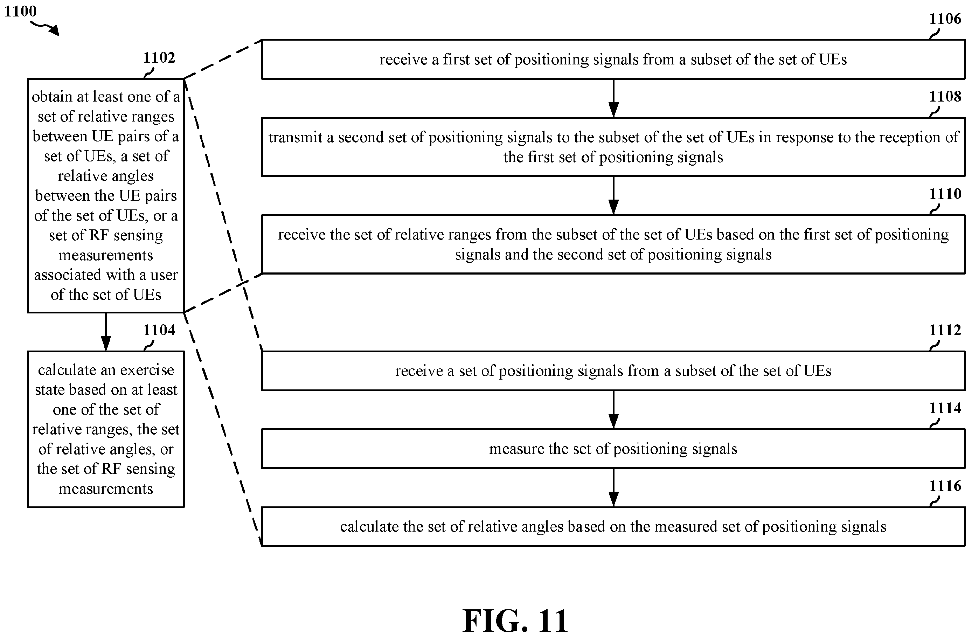

FIG. 11 is a flowchart 1100 of a method of wireless communication. The method may be performed by a UE (e.g., the UE 104, the UE 350, the UE 602, the UE 604, the UE 606, the UE 608, the UE 702, the UE 704, the UE 706; the equipment 610; the AP 612; the wireless device 402, the wireless device 404, the wireless device 406, the wireless device 502, the wireless device 504, the wireless device 506, the wireless device 508; the apparatus 1604). At 1102, the UE may obtain at least one of a set of relative ranges between UE pairs of a set of UEs, a set of relative angles between the UE pairs of the set of UEs, or a set of RF sensing measurements associated with a user of the set of UEs. For example, 1102 may be performed by the UE 602 in FIG. 6, which may obtain at least one of a set of relative ranges between UE pairs (e.g., between the UE 602 and the UE 604) of the set of UEs (e.g., the UE 602, the UE 604, the UE 606, and the UE 608), a set of relative angles between the UE pairs of the set of UEs, or a set of RF sensing measurements associated with the user 600 of the set of UEs. Moreover, 1102 may be performed by the component 198 in FIG. 16.

At 1104, the UE may calculate an exercise state based on at least one of the set of relative ranges, the set of relative angles, or the set of RF sensing measurements. For example, 1104 may be performed by the UE 602 in FIG. 6, which may calculate an exercise state of the user 600 of the UE 602, the UE 604, the UE 606, and the UE 608 based on at least one of the set of relative ranges, the set of relative angles, or the set of RF sensing measurements. Moreover, 1104 may be performed by the component 198 in FIG. 16.

At 1106, the UE may obtain the set of relative ranges between UE pairs of the set of UEs by receiving a first set of positioning signals from a subset of the set of UEs. For example, 1106 may be performed by the UE 602 in FIG. 6, which may receive a first set of positioning signals from the UE 604, the UE 606, and the UE 608 of the set of UEs that include the UE 602, the UE 604, the UE 606, the UE 608, the equipment 610, and the AP 612. Moreover, 1106 may be performed by the component 198 in FIG. 16.

At 1108, the UE may obtain the set of relative ranges between UE pairs of the set of UEs by transmitting a second set of positioning signals to the subset of the set of UEs in response to the reception of the first set of positioning signals. For example, 1108 may be performed by the UE 602 in FIG. 6, which may transmit a second set of positioning signals to the UE 604, the UE 606, and the UE 608 of the set of UEs in response to the reception of the first set of positioning signals. Moreover, 1108 may be performed by the component 198 in FIG. 16.

At 1110, the UE may obtain the set of relative ranges between UE pairs of the set of UEs by receiving the set of relative ranges from the subset of the set of UEs based on the first set of positioning signals and the second set of positioning signals. For example, 1110 may be performed by the UE 602 in FIG. 6, which may receive the set of relative ranges from the UE 604, the UE 606, and the UE 608 of the set of UEs based on the first set of positioning signals and the second set of positioning signals (e.g., an RTT measurement or a RTOA measurement). Moreover, 1110 may be performed by the component 198 in FIG. 16.

At 1112, the UE may obtain the set of relative ranges between UE pairs of the set of UEs by receiving a set of positioning signals from a subset of the set of UEs. For example, 1112 may be performed by the UE 602 in FIG. 6, which may receive a set of positioning signals from the UE 604, the UE 606, and the UE 608 of the set of UEs that include the UE 602, the UE 604, the UE 606, the UE 608, the equipment 610, and the AP 612. Moreover, 1112 may be performed by the component 198 in FIG. 16.

At 1114, the UE may obtain the set of relative ranges between UE pairs of the set of UEs by measuring the set of positioning signals. For example, 1114 may be performed by the UE 602 in FIG. 6, which may measure the set of positioning signals. Moreover, 1114 may be performed by the component 198 in FIG. 16.

At 1116, the UE may obtain the set of relative ranges between UE pairs of the set of UEs by calculating the set of relative angles based on the measured set of positioning signals. For example, 1116 may be performed by the UE 602 in FIG. 6, which may calculate the set of relative angles between the UE 602 and the UE 604, the UE 606, and the UE 608 based on the measured set of positioning signals. Moreover, 1116 may be performed by the component 198 in FIG. 16.

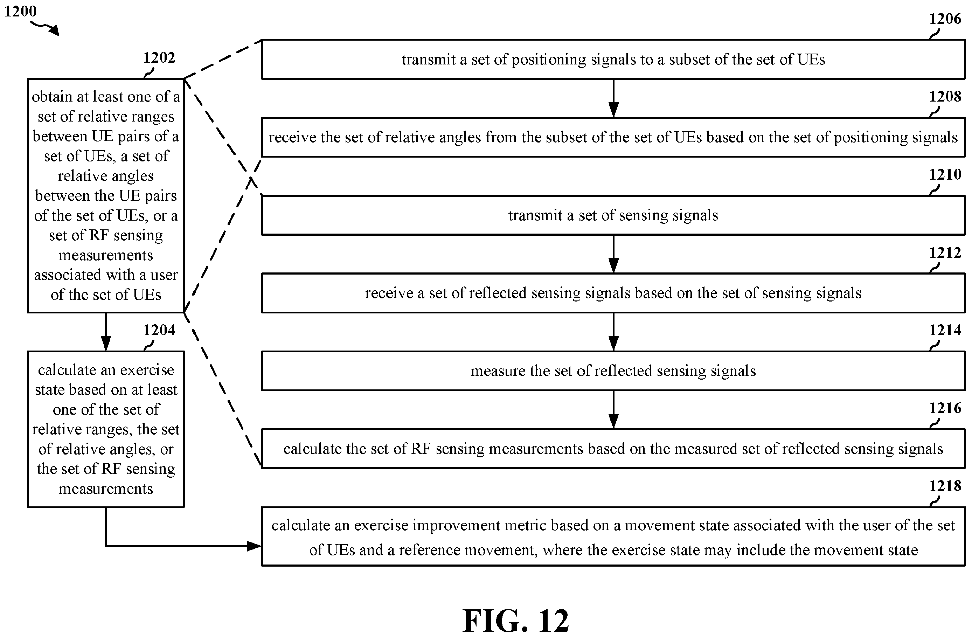

FIG. 12 is a flowchart 1200 of a method of wireless communication. The method may be performed by a UE (e.g., the UE 104, the UE 350, the UE 602, the UE 604, the UE 606, the UE 608, the UE 702, the UE 704, the UE 706; the equipment 610; the AP 612; the wireless device 402, the wireless device 404, the wireless device 406, the wireless device 502, the wireless device 504, the wireless device 506, the wireless device 508; the apparatus 1604). At 1202, the UE may obtain at least one of a set of relative ranges between UE pairs of a set of UEs, a set of relative angles between the UE pairs of the set of UEs, or a set of RF sensing measurements associated with a user of the set of UEs. For example, 1202 may be performed by the UE 602 in FIG. 6, which may obtain at least one of a set of relative ranges between UE pairs (e.g., between the UE 602 and the UE 604) of the set of UEs (e.g., the UE 602, the UE 604, the UE 606, and the UE 608), a set of relative angles between the UE pairs of the set of UEs, or a set of RF sensing measurements associated with the user 600 of the set of UEs. Moreover, 1202 may be performed by the component 198 in FIG. 16.

At 1204, the UE may calculate an exercise state based on at least one of the set of relative ranges, the set of relative angles, or the set of RF sensing measurements. For example, 1204 may be performed by the UE 602 in FIG. 6, which may calculate an exercise state of the user 600 of the UE 602, the UE 604, the UE 606, and the UE 608 based on at least one of the set of relative ranges, the set of relative angles, or the set of RF sensing measurements. Moreover, 1204 may be performed by the component 198 in FIG. 16.

At 1206, the UE may obtain the set of relative angles between the UE pairs of the set of UEs by transmitting a set of positioning signals to a subset of the set of UEs. For example, 1206 may be performed by the UE 602 in FIG. 6, which may transmit a set of positioning signals to the UE 604, the UE 606, and the UE 608 of the set of UEs that include the UE 602, the UE 604, the UE 606, the UE 608, the equipment 610, and the AP 612. Moreover, 1206 may be performed by the component 198 in FIG. 16.

At 1208, the UE may obtain the set of relative angles between the UE pairs of the set of UEs by receiving the set of relative angles from the subset of the set of UEs based on the set of positioning signals. For example, 1208 may be performed by the UE 602 in FIG. 6, which may receive the set of relative angles from the UE 604, the UE 606, and the UE 608 of the set of UEs based on the set of positioning signals. Moreover, 1208 may be performed by the component 198 in FIG. 16.

At 1210, the UE may obtain the set of RF sensing measurements between the UE pairs of the set of UEs by transmitting a set of sensing signals. For example, 1210 may be performed by the UE 602 in FIG. 6, which may transmit a set of sensing signals. Moreover, 1210 may be performed by the component 198 in FIG. 16.

At 1212, the UE may obtain the set of RF sensing measurements between the UE pairs of the set of UEs by receiving a set of reflected sensing signals based on the set of sensing signals. For example, 1212 may be performed by the UE 602 in FIG. 6, which may receive a set of reflected sensing signals based on the set of sensing signals. The reflected sensing signals may be reflected by UE 604, the UE 606, the UE 608, the equipment 610, and/or the AP 612. The reflected sensing signals may be reflected by parts of the body of the user 600. The reflected sensing signals may be reflected off of other objects about the user 600. Moreover, 1212 may be performed by the component 198 in FIG. 16.

At 1214, the UE may obtain the set of RF sensing measurements between the UE pairs of the set of UEs by measuring the set of reflected sensing signals. For example, 1214 may be performed by the UE 602 in FIG. 6, which may measure the set of reflected sensing signals. Moreover, 1214 may be performed by the component 198 in FIG. 16.

At 1216, the UE may obtain the set of RF sensing measurements between the UE pairs of the set of UEs by calculating the set of RF sensing measurements based on the measured set of reflected sensing signals. For example, 1216 may be performed by the UE 602 in FIG. 6, which may calculate the set of RF sensing measurements based on the measured set of reflected sensing signals. The set of RF sensing measurements may be used to determine the distance between the UE 602 and objects about the user 600, such as the UE 604, the UE 606, the UE 608, the equipment 610, the AP 612, and/or parts of the body of the user 600. The set of RF sensing measurements may be used to determine an angle between the UE 602 and objects about the user 600, such as the UE 604, the UE 606, the UE 608, the equipment 610, the AP 612, and/or parts of the body of the user 600. Moreover, 1216 may be performed by the component 198 in FIG. 16.

At 1218, the UE may calculate an exercise improvement metric based on a movement state associated with the user of the set of UEs and a reference movement, where the exercise state may include the movement state. For example, 1218 may be performed by the UE 602 in FIG. 6, which may calculate an exercise improvement metric based on a movement state associated with the user 600 of the set of UEs and a reference movement. The exercise state may include the movement state of the user 600. The exercise improvement metric may be based on a difference between the movement state and the reference movement. For example, if the reference movement is of a person raising their arm by a first distance, and the movement state is of the user 600 raising their arm by a second distance less than the first distance, the exercise improvement metric may be based on a difference between the first distance and the second distance. Moreover, 1218 may be performed by the component 198 in FIG. 16.

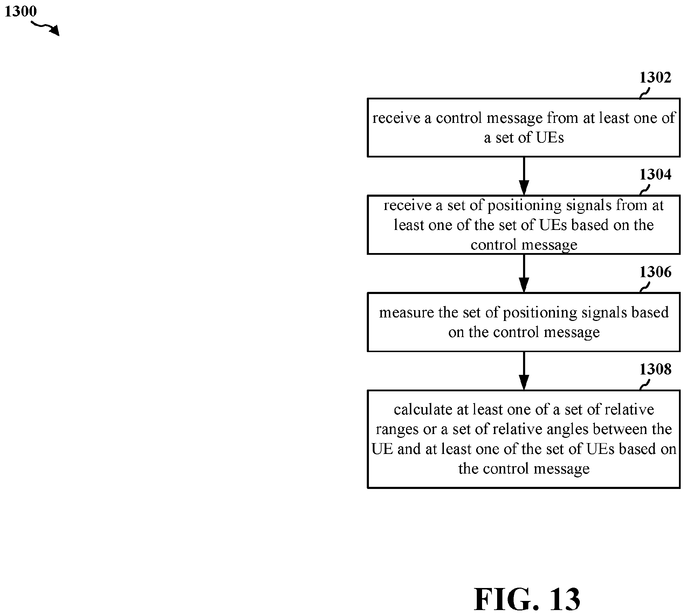

FIG. 13 is a flowchart 1300 of a method of wireless communication. The method may be performed by a UE (e.g., the UE 104, the UE 350, the UE 602, the UE 604, the UE 606, the UE 608, the UE 702, the UE 704, the UE 706; the equipment 610; the AP 612; the wireless device 402, the wireless device 404, the wireless device 406, the wireless device 502, the wireless device 504, the wireless device 506, the wireless device 508; the apparatus 1604). At 1302, the UE may receive a control message from at least one of a set of UEs. For example, 1302 may be performed by the UE 704 in FIG. 7, which may receive the control message 708 from the UE 702 of the set of UEs that may include the UE 702, the UE 704, and the UE 706. Moreover, 1302 may be performed by the component 199 in FIG. 16.

At 1304, the UE may receive a set of positioning signals from at least one of the set of UEs based on the control message. For example, 1304 may be performed by the UE 704 in FIG. 7, which may receive a set of positioning signals as the initiation message 712, or a set of positioning signals from the UE 706, based on the control message 708. Moreover, 1304 may be performed by the component 199 in FIG. 16.

At 1306, the UE may measure the set of positioning signals based on the control message. For example, 1306 may be performed by the UE 704 in FIG. 7, which may measure the set of positioning signals based on the control message 708 by performing positioning in the received positioning signals. Moreover, 1306 may be performed by the component 199 in FIG. 16.

At 1308, the UE may calculate at least one of a set of relative ranges or a set of relative angles between the UE and at least one of the set of UEs based on the control message. For example, 1308 may be performed by the UE 704 in FIG. 7, which may calculate at least one of a set of relative ranges or a set of relative angles between the UE 704 and at least one of the UE 702 or the UE 706 based on the control message 708. Moreover, 1308 may be performed by the component 199 in FIG. 16.

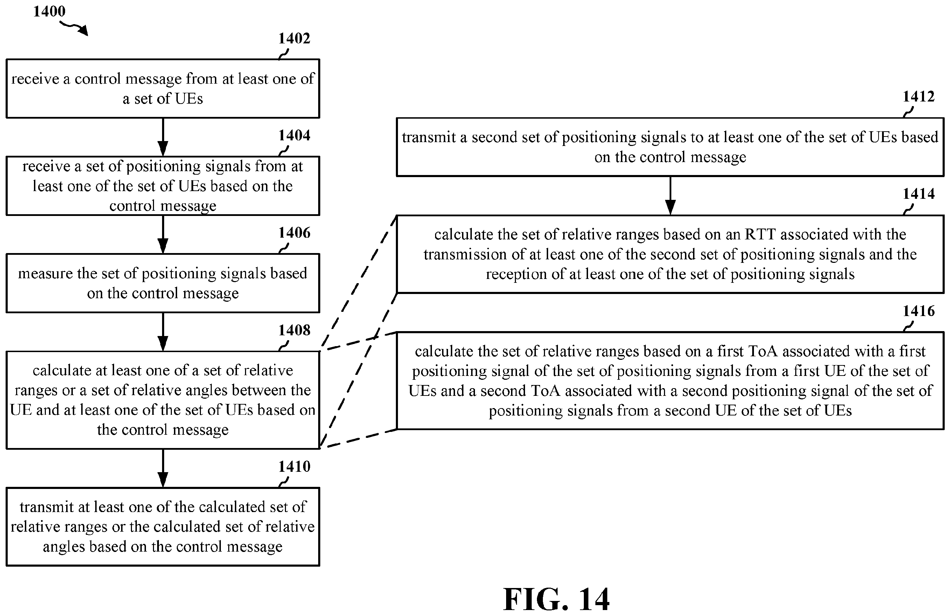

FIG. 14 is a flowchart 1400 of a method of wireless communication. The method may be performed by a UE (e.g., the UE 104, the UE 350, the UE 602, the UE 604, the UE 606, the UE 608, the UE 702, the UE 704, the UE 706; the equipment 610; the AP 612; the wireless device 402, the wireless device 404, the wireless device 406, the wireless device 502, the wireless device 504, the wireless device 506, the wireless device 508; the apparatus 1604). At 1402, the UE may receive a control message from at least one of a set of UEs. For example, 1402 may be performed by the UE 704 in FIG. 7, which may receive the control message 708 from the UE 702 of the set of UEs that may include the UE 702, the UE 704, and the UE 706. Moreover, 1402 may be performed by the component 199 in FIG. 16.

At 1404, the UE may receive a set of positioning signals from at least one of the set of UEs based on the control message. For example, 1404 may be performed by the UE 704 in FIG. 7, which may receive a set of positioning signals as the initiation message 712, or a set of positioning signals from the UE 706, based on the control message 708. Moreover, 1404 may be performed by the component 199 in FIG. 16.

At 1406, the UE may measure the set of positioning signals based on the control message. For example, 1406 may be performed by the UE 704 in FIG. 7, which may measure the set of positioning signals based on the control message 708 by performing positioning in the received positioning signals. Moreover, 1406 may be performed by the component 199 in FIG. 16.

At 1408, the UE may calculate at least one of a set of relative ranges or a set of relative angles between the UE and at least one of the set ofUEs based on the control message. For example, 1408 may be performed by the UE 704 in FIG. 7, which may calculate at least one of a set of relative ranges or a set of relative angles between the UE 704 and at least one of the UE 702 or the UE 706 based on the control message 708. Moreover, 1408 may be performed by the component 199 in FIG. 16.

At 1410, the UE may transmit at least one of the calculated set of relative ranges or the calculated set of relative angles based on the control message. For example, 1410 may be performed by the UE 704 in FIG. 7, which may transmit at least one of the calculated set of relative ranges or the calculated set of relative angles based on the control message. This information may be transmitted as the response message 716. Moreover, 1410 may be performed by the component 199 in FIG. 16.

At 1412, the UE may transmit a second set of positioning signals to at least one of the set of UEs based on the control message. For example, 1412 may be performed by the UE 704 in FIG. 7, which may transmit a second set of positioning signals as the response message 716 to the UE 702 and/or the UE 706 based on the control message 708. Moreover, 1412 may be performed by the component 199 in FIG. 16.

At 1414, the UE may calculate the set of relative ranges by calculating the set of relative ranges based on an RTT associated with the transmission of at least one of the second set of positioning signals and the reception of at least one of the set of positioning signals. For example, 1414 may be performed by the UE 704 in FIG. 7, which may calculate the relative range between the UE 704 and the UE 702, or between the UE 704 and the UE 706 based on an RTT associated with the transmission of the response message 716 and the reception of the initiation message 712 or a response from the response message 716. Moreover, 1414 may be performed by the component 199 in FIG. 16.

At 1416, the UE may calculate the set of relative ranges by calculating the set of relative ranges based on a first ToA associated with a first positioning signal of the set of positioning signals from a first UE of the set of UEs and a second ToA associated with a second positioning signal of the set of positioning signals from a second UE of the set of UEs. For example, 1416 may be performed by the UE 704 in FIG. 7, which may calculate the set of relative ranges based on a first ToA associated with a first positioning signal of the initiation message 712 from the UE 702 of the set of UEs and a second ToA associated with a second positioning signal of the initiation message 712 from the UE 706 of the set of UEs. Moreover, 1416 may be performed by the component 199 in FIG. 16.

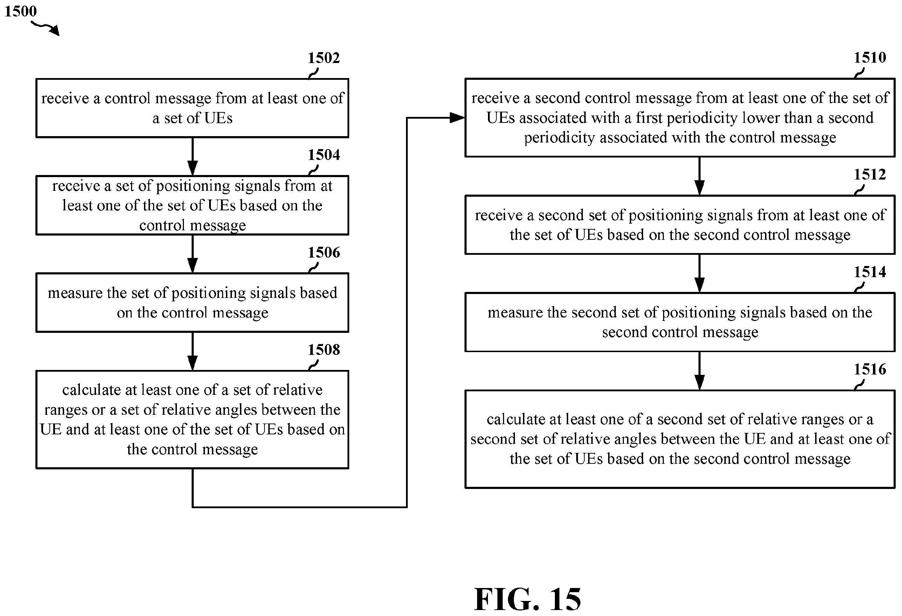

FIG. 15 is a flowchart 1500 of a method of wireless communication. The method may be performed by a UE (e.g., the UE 104, the UE 350, the UE 602, the UE 604, the UE 606, the UE 608, the UE 702, the UE 704, the UE 706; the equipment 610; the AP 612; the wireless device 402, the wireless device 404, the wireless device 406, the wireless device 502, the wireless device 504, the wireless device 506, the wireless device 508; the apparatus 1604). At 1502, the UE may receive a control message from at least one of a set of UEs. For example, 1502 may be performed by the UE 704 in FIG. 7, which may receive the control message 708 from the UE 702 of the set of UEs that may include the UE 702, the UE 704, and the UE 706. Moreover, 1502 may be performed by the component 199 in FIG. 16.

At 1504, the UE may receive a set of positioning signals from at least one of the set of UEs based on the control message. For example, 1504 may be performed by the UE 704 in FIG. 7, which may receive a set of positioning signals as the initiation message 712, or a set of positioning signals from the UE 706, based on the control message 708. Moreover, 1504 may be performed by the component 199 in FIG. 16.

At 1506, the UE may measure the set of positioning signals based on the control message. For example, 1506 may be performed by the UE 704 in FIG. 7, which may measure the set of positioning signals based on the control message 708 by performing positioning in the received positioning signals. Moreover, 1506 may be performed by the component 199 in FIG. 16.

At 1508, the UE may calculate at least one of a set of relative ranges or a set of relative angles between the UE and at least one of the set of UEs based on the control message. For example, 1508 may be performed by the UE 704 in FIG. 7, which may calculate at least one of a set of relative ranges or a set of relative angles between the UE 704 and at least one of the UE 702 or the UE 706 based on the control message 708. Moreover, 1508 may be performed by the component 199 in FIG. 16.

At 1510, the UE may receive a second control message from at least one of the set of UEs associated with a first periodicity lower than a second periodicity associated with the control message. For example, 1510 may be performed by the UE 704 in FIG. 7, which may receive a second control message as the control message 708 from the UE 702. The control message 708 received later may be an update of the control message 708 received previously. The control message 708 received later may be associated with a lower periodicity than a periodicity associated with the control message 708 received previously. Moreover, 1510 may be performed by the component 199 in FIG. 16.

At 1512, the UE may receive a second set of positioning signals from at least one of the set of UEs based on the second control message. For example, 1512 may be performed by the UE 704 in FIG. 7, which may receive a second set of positioning signals as the initiation message 712 from the UE 702 based on the control message 708 received later. Moreover, 1512 may be performed by the component 199 in FIG. 16.

At 1514, the UE may measure the second set of positioning signals based on the second control message. For example, 1514 may be performed by the UE 704 in FIG. 7, which may measure the second set of positioning signals based on the control message 708 received later. Moreover, 1514 may be performed by the component 199 in FIG. 16.

At 1516, the UE may calculate at least one of a second set of relative ranges or a second set of relative angles between the UE and at least one of the set of UEs based on the second control message. For example, 1516 may be performed by the UE 704 in FIG. 7, which may calculate at least one of a second set of relative ranges or a second set of relative angles between the UE 704 and the UE 702 based on the control message 708 received later. As the control message 708 received later may have a lower periodicity than the control message 708 received earlier, the later calculation may have a greater granularity due to the lower periodicity. Moreover, 1516 may be performed by the component 199 in FIG. 16.

FIG. 16 is a diagram 1600 illustrating an example of a hardware implementation for an apparatus 1604. The apparatus 1604 may be a UE, a component of a UE, or may implement UE functionality. In some aspects, the apparatus1104 may include a cellular baseband processor 1624 (also referred to as a modem) coupled to one or more transceivers 1622 (e.g., cellular RF transceiver). The cellular baseband processor 1624 may include on-chip memory 1624'. In some aspects, the apparatus 1604 may further include one or more subscriber identity modules (SIM) cards 1620 and an application processor 1606 coupled to a secure digital (SD) card 1608 and a screen 1610. The application processor 1606 may include on-chip memory 1606'. In some aspects, the apparatus 1604 may further include a Bluetooth module 1612, a WLAN module 1614, an SPS module 1616 (e.g., GNSS module), one or more sensor modules 1618 (e.g., barometric pressure sensor / altimeter; motion sensor such as inertial measurement unit (IMU), gyroscope, and/or accelerometer(s); light detection and ranging (LIDAR), radio assisted detection and ranging (RADAR), sound navigation and ranging (SONAR), magnetometer, audio and/or other technologies used for positioning), additional memory modules 1626, a power supply 1630, and/or a camera 1632. The Bluetooth module 1612, the WLAN module 1614, and the SPS module 1616 may include an on-chip transceiver (TRX) (or in some cases, just a receiver (RX)). The Bluetooth module 1612, the WLAN module 1614, and the SPS module 1616 may include their own dedicated antennas and/or utilize the antennas 1680 for communication. The cellular baseband processor 1624 communicates through the transceiver(s) 1622 via one or more antennas 1680 with the UE 104 and/or with an RU associated with a network entity 1602. The cellular baseband processor 1624 and the application processor 1606 may each include a computer-readable medium / memory 1624', 1606', respectively. The additional memory modules 1626 may also be considered a computer-readable medium / memory. Each computer-readable medium / memory 1624', 1606', 1626 may be non-transitory. The cellular baseband processor 1624 and the application processor 1606 are each responsible for general processing, including the execution of software stored on the computer-readable medium / memory. The software, when executed by the cellular baseband processor 1624 / application processor 1606, causes the cellular baseband processor 1624 / application processor 1606 to perform the various functions described *supra.* The computer-readable medium / memory may also be used for storing data that is manipulated by the cellular baseband processor 1624 / application processor 1606 when executing software. The cellular baseband processor 1624 / application processor 1606 may be a component of the UE 350 and may include the memory 360 and/or at least one of the TX processor 368, the RX processor 356, and the controller/processor 359. In one configuration, the apparatus 1604 may be a processor chip (modem and/or application) and include just the cellular baseband processor 1624 and/or the application processor 1606, and in another configuration, the apparatus 1604 may be the entire UE (e.g., see UE 350 of FIG. 3) and include the additional modules of the apparatus 1604.

As discussed *supra,* the component 198 may be configured to obtain at least one of a set of relative ranges between UE pairs of a set of UEs, a set of relative angles between the UE pairs of the set of UEs, or a set of RF sensing measurements associated with a user of the set of UEs. The component 198 may be configured to calculate an exercise state based on at least one of the set of relative ranges, the set of relative angles, or the set of RF sensing measurements. The component 198 may be within the cellular baseband processor 1624, the application processor 1606, or both the cellular baseband processor 1624 and the application processor 1606. The component 198 may be one or more hardware components specifically configured to carry out the stated processes/algorithm, implemented by one or more processors configured to perform the stated processes/algorithm, stored within a computer-readable medium for implementation by one or more processors, or some combination thereof. As shown, the apparatus 1604 may include a variety of components configured for various functions. In one configuration, the apparatus 1604, and in particular the cellular baseband processor 1624 and/or the application processor 1606, may include means for obtaining at least one of a set of relative ranges between UE pairs of a set of UEs, a set of relative angles between the UE pairs of the set of UEs, or a set of RF sensing measurements associated with a user of the set of UEs. The apparatus 1604 may include means for calculating an exercise state based on at least one of the set of relative ranges, the set of relative angles, or the set of RF sensing measurements. The set of UEs may include the UE. The apparatus 1604 may include means for obtaining the set of relative ranges by transmitting a first set of positioning signals to a subset of the set of UEs, receiving a second set of positioning signals from the subset of the set of UEs in response to the transmission of the first set of positioning signals, measuring the second set of positioning signals, and calculating the set of relative ranges based on the measured second set of positioning signals. The apparatus 1604 may include means for calculating the set of relative ranges by calculating the set of relative ranges based on a first ToA associated with a first UE of the subset of the set of UEs and a second ToA associated with a second UE of the subset of the set of UEs. The apparatus 1604 may include means for calculating the set of relative ranges by calculating the set of relative ranges based on an RTT associated with the transmission of at least one of the first set of positioning signals and the reception of at least one of the second set of positioning signals. The apparatus 1604 may include means for obtaining the set of relative ranges by receiving a first set of positioning signals from a subset of the set of UEs, transmitting a second set of positioning signals to the subset of the set of UEs in response to the reception of the first set of positioning signals, and receiving the set of relative ranges from the subset of the set of UEs based on the first set of positioning signals and the second set of positioning signals. The apparatus 1604 may include means for obtaining the set of relative angles by receiving a set of positioning signals from a subset of the set of UEs, measuring the set of positioning signals, and calculating the set of relative angles based on the measured set of positioning signals. The set of relative angles may include an AoA associated with a positioning signal of the set of positioning signals and a corresponding UE of the subset of the set of UEs. The apparatus 1604 may include means for obtaining the set of relative angles by transmitting a set of positioning signals to a subset of the set of UEs, and receiving the set of relative angles from the subset of the set of UEs based on the set of positioning signals. The apparatus 1604 may include means for obtaining the set of RF sensing measurements associated with the user of the set of UEs by transmitting a set of sensing signals, receiving a set of reflected sensing signals based on the set of sensing signals, measuring the set of reflected sensing signals, and calculating the set of RF sensing measurements based on the measured set of reflected sensing signals. The sensing signals may be transmitted at parts of the user associated with the set of UEs. The exercise state may include at least one of a movement state associated with the user of the set of UEs or a type of exercise associated with the user. The apparatus 1604 may include means for calculating an exercise improvement metric based on the movement state and an idealized movement. The apparatus 1604 may include means for transmitting a control message to a subset of the set of UEs. The apparatus 1604 may include means for obtaining the set of relative ranges or obtaining the set of relative angles in response to the transmission of the control message to the subset of the set of UEs. The control message may be an RCM. The apparatus 1604 may include means for calculating the exercise state by calculating a movement metric associated with the user associated with the set of UEs, wherein the movement metric is greater than a threshold value. The apparatus 1604 may include means for transmitting a second control message to the subset of the set of UEs associated with a first periodicity lower than a second periodicity associated with the control message. The means may be the component 198 of the apparatus 1604 configured to perform the functions recited by the means. As described *supra*, the apparatus 1604 may include the TX processor 368, the RX processor 356, and the controller/processor 359. As such, in one configuration, the means may be the TX processor 368, the RX processor 356, and/or the controller/processor 359 configured to perform the functions recited by the means.

As discussed *supra,* the component 199 may be configured to receive a control message from at least one of a set of UEs. The component 199 may be configured to receive a set of positioning signals from at least one of the set of UEs based on the control message. The component 199 may be configured to measure the set of positioning signals based on the control message. The component 199 may be configured to calculate at least one of a set of relative ranges or a set of relative angles between the UE and at least one of the set of UEs based on the control message. The component 199 may be within the cellular baseband processor 1624, the application processor 1606, or both the cellular baseband processor 1624 and the application processor 1606. The component 199 may be one or more hardware components specifically configured to carry out the stated processes/algorithm, implemented by one or more processors configured to perform the stated processes/algorithm, stored within a computer-readable medium for implementation by one or more processors, or some combination thereof. As shown, the apparatus 1604 may include a variety of components configured for various functions. In one configuration, the apparatus 1604, and in particular the cellular baseband processor 1624 and/or the application processor 1606, may include means for receiving a control message from at least one of a set of UEs. The control message may be an RCM. The apparatus 1604 may include means for receiving a set of positioning signals from at least one of the set of UEs based on the control message. The apparatus 1604 may include means for measuring the set of positioning signals based on the control message. The apparatus 1604 may include means for calculating at least one of a set of relative ranges or a set of relative angles between the UE and at least one of the set of UEs based on the control message. The control message may include at least one of a set of resources associated with the set of positioning signals, or an indication of a set of transmission times associated with the set of positioning signals. The apparatus 1604 may include means for transmitting at least one of the calculated set of relative ranges or the calculated set of relative angles based on the control message. The apparatus 1604 may include means for calculating the set of relative ranges by calculating the set of relative ranges based on a first ToA associated with a first positioning signal of the set of positioning signals from a first UE of the set of UEs and a second ToA associated with a second positioning signal of the set of positioning signals from a second UE of the set of UEs. The apparatus 1604 may include means for transmitting a second set of positioning signals to at least one of the set of UEs based on the control message. The apparatus 1604 may include means for calculating the set of relative ranges by calculating the set of relative ranges based on a RTT associated with the transmission of at least one of the second set of positioning signals and the reception of at least one of the set of positioning signals. The set of relative angles may include an AoA associated with a positioning signal of the set of positioning signals and a corresponding UE of the set of UEs. The apparatus 1604 may include means for receiving a second control message from at least one of the set of UEs associated with a first periodicity lower than a second periodicity associated with the control message. The apparatus 1604 may include means for receiving a second set of positioning signals from at least one of the set of UEs based on the second control message. The apparatus 1604 may include means for measuring the second set of positioning signals based on the second control message. The apparatus 1604 may include means for calculating at least one of a second set of relative ranges or a second set of relative angles between the UE and at least one of the set of UEs based on the second control message. The means may be the component 199 of the apparatus 1604 configured to perform the functions recited by the means. As described *supra,* the apparatus 1604 may include the TX processor 368, the RX processor 356, and the controller/processor 359. As such, in one configuration, the means may be the TX processor 368, the RX processor 356, and/or the controller/processor 359 configured to perform the functions recited by the means.

It is understood that the specific order or hierarchy of blocks in the processes / flowcharts disclosed is an illustration of example approaches. Based upon design preferences, it is understood that the specific order or hierarchy of blocks in the processes / flowcharts may be rearranged. Further, some blocks may be combined or omitted. The accompanying method claims present elements of the various blocks in a sample order, and are not limited to the specific order or hierarchy presented.

The previous description is provided to enable any person skilled in the art to practice the various aspects described herein. Various modifications to these aspects will be readily apparent to those skilled in the art, and the generic principles defined herein may be applied to other aspects. Thus, the claims are not limited to the aspects described herein, but are to be accorded the full scope consistent with the language claims. Reference to an element in the singular does not mean "one and only one" unless specifically so stated, but rather "one or more." Terms such as "if," "when," and "while" do not imply an immediate temporal relationship or reaction. That is, these phrases, e.g., "when," do not imply an immediate action in response to or during the occurrence of an action, but simply imply that if a condition is met then an action will occur, but without requiring a specific or immediate time constraint for the action to occur. The word "exemplary" is used herein to mean "serving as an example, instance, or illustration." Any aspect described herein as "exemplary" is not necessarily to be construed as preferred or advantageous over other aspects. Unless specifically stated otherwise, the term "some" refers to one or more. Combinations such as "at least one of A, B, or C," "one or more of A, B, or C," "at least one of A, B, and C," "one or more of A, B, and C," and "A, B, C, or any combination thereof" include any combination of A, B, and/or C, and may include multiples of A, multiples of B, or multiples of C. Specifically, combinations such as "at least one of A, B, or C," "one or more of A, B, or C," "at least one of A, B, and C," "one or more of A, B, and C," and "A, B, C, or any combination thereof" may be A only, B only, C only, A and B, A and C, B and C, or A and B and C, where any such combinations may contain one or more member or members of A, B, or C. Sets should be interpreted as a set of elements where the elements number one or more. Accordingly, for a set of X, X would include one or more elements. If a first apparatus receives data from or transmits data to a second apparatus, the data may be received/transmitted directly between the first and second apparatuses, or indirectly between the first and second apparatuses through a set of apparatuses. A device configured to "output" data, such as a transmission, signal, or message, may transmit the data, for example with a transceiver, may send the data to a device that transmits the data, or may provide the data to a component of the device. A device configured to "obtain" data, such as a transmission, signal, or message, may receive the data, for example with a transceiver, may obtain the data from a device that receives the data, or may obtain the data from a component of the device. All structural and functional equivalents to the elements of the various aspects described throughout this disclosure that are known or later come to be known to those of ordinary skill in the art are expressly incorporated herein by reference and are encompassed by the claims. Moreover, nothing disclosed herein is dedicated to the public regardless of whether such disclosure is explicitly recited in the claims. The words "module," "mechanism," "element," "device," and the like may not be a substitute for the word "means." As such, no claim element is to be construed as a means plus function unless the element is expressly recited using the phrase "means for."

As used herein, the phrase "based on" shall not be construed as a reference to a closed set of information, one or more conditions, one or more factors, or the like. In other words, the phrase "based on A" (where "A" may be information, a condition, a factor, or the like) shall be construed as "based at least on A" unless specifically recited differently.

The following aspects are illustrative only and may be combined with other aspects or teachings described herein, without limitation.

Aspect 1 is a method of wireless communication at a user equipment (UE), wherein the method comprises obtaining at least one of a set of relative ranges between UE pairs of a set of UEs, a set of relative angles between the UE pairs of the set of UEs, or a set of radio frequency (RF) sensing measurements associated with a user of the set of UEs. The method further comprises calculating an exercise state based on at least one of the set of relative ranges, the set of relative angles, or the set of RF sensing measurements.

Aspect 2 is the method of aspect 1, wherein the set of UEs comprises the UE.

Aspect 3 is the method of aspect 2, wherein obtaining the set of relative ranges comprises transmitting a first set of positioning signals to a subset of the set of UEs, receiving a second set of positioning signals from the subset of the set of UEs in response to the transmission of the first set of positioning signals, measuring the second set of positioning signals, and calculating the set of relative ranges based on the measured second set of positioning signals.

Aspect 4 is the method of aspect 3, wherein calculating the set of relative ranges comprises calculating the set of relative ranges based on a first time of arrival (ToA) associated with a first UE of the subset of the set of UEs and a second ToA associated with a second UE of the subset of the set of UEs.

Aspect 5 is the method of either of aspects 3 or 4, wherein calculating the set of relative ranges comprises calculating the set of relative ranges based on a round trip time (RTT) associated with the transmission of at least one of the first set of positioning signals and the reception of at least one of the second set of positioning signals.

Aspect 6 is the method of any of aspects 2 to 5, wherein obtaining the set of relative ranges comprises receiving a first set of positioning signals from a subset of the set of UEs, transmitting a second set of positioning signals to the subset of the set of UEs in response to the reception of the first set of positioning signals, and receiving the set of relative ranges from the subset of the set of UEs based on the first set of positioning signals and the second set of positioning signals.

Aspect 7 is the method of any of aspects 1 to 6, wherein obtaining the set of relative angles comprises receiving a set of positioning signals from a subset of the set of UEs, measuring the set of positioning signals, and calculating the set of relative angles based on the measured set of positioning signals.

Aspect 8 is the method of aspect 7, wherein the set of relative angles comprises an angle of arrival (AoA) associated with a positioning signal of the set of positioning signals and a corresponding UE of the subset of the set of UEs.

Aspect 9 is the method of any of aspects 1 to 8, wherein obtaining the set of relative angles comprises transmitting a set of positioning signals to a subset of the set of UEs, and receiving the set of relative angles from the subset of the set of UEs based on the set of positioning signals.

Aspect 10 is the method of any of aspects 1 to 9, wherein obtaining the set of RF sensing measurements associated with the user of the set of UEs comprises transmitting a set of sensing signals, receiving a set of reflected sensing signals based on the set of sensing signals, measuring the set of reflected sensing signals, and calculating the set of RF sensing measurements based on the measured set of reflected sensing signals. The sensing signals may be transmitted at parts of the user associated with the set of UEs.

Aspect 11 is the method of any of aspects 1 to 10, wherein the exercise state comprises at least one of a movement state associated with the user of the set of UEs or a type of exercise associated with the user.

Aspect 12 is the method of any of aspects 1 to 11, wherein the method comprises calculating an exercise improvement metric based on the movement state and an idealized movement.

Aspect 13 is the method of any of aspects 1 to 12, wherein the method comprises transmitting a control message to a subset of the set of UEs, wherein obtaining the set of relative ranges or obtaining the set of relative angles is in response to the transmission of the control message to the subset of the set of UEs. The control message may be a ranging control message (RCM).

Aspect 14 is the method of aspect 13, wherein calculating the exercise state comprises calculating a movement metric associated with the user associated with the set of UEs, wherein the movement metric is greater than a threshold value. The method further comprises transmitting a second control message to the subset of the set of UEs associated with a first periodicity lower than a second periodicity associated with the control message.

Aspect 15 is a method of wireless communication at a user equipment (UE), wherein the method comprises receiving a control message from at least one of a set of UEs. The control message may be a ranging control message (RCM). The method further comprises receiving a set of positioning signals from at least one of the set of UEs based on the control message. The method further comprises measuring the set of positioning signals based on the control message. The method further comprises calculating at least one of a set of relative ranges or a set of relative angles between the UE and at least one of the set of UEs based on the control message.

Aspect 16 is the method of aspect 15, wherein the control message comprises at least one of a set of resources associated with the set of positioning signals, or an indication of a set of transmission times associated with the set of positioning signals.

Aspect 17 is the method of either of aspects 15 or 16, wherein the method further comprises transmitting at least one of the calculated set of relative ranges or the calculated set of relative angles based on the control message.

Aspect 18 is the method of any of aspects 15 to 17, wherein calculating the set of relative ranges comprises calculating the set of relative ranges based on a first time of arrival (ToA) associated with a first positioning signal of the set of positioning signals from a first UE of the set of UEs and a second ToA associated with a second positioning signal of the set of positioning signals from a second UE of the set of UEs.

Aspect 19 is the method of any of aspects 15 to 18, wherein the method further comprises transmitting a second set of positioning signals to at least one of the set of UEs based on the control message, wherein calculating the set of relative ranges comprises calculating the set of relative ranges based on a round trip time (RTT) associated with the transmission of at least one of the second set of positioning signals and the reception of at least one of the set of positioning signals.

Aspect 20 is the method of any of aspects 15 to 19, wherein the set of relative angles comprises an angle of arrival (AoA) associated with a positioning signal of the set of positioning signals and a corresponding UE of the set of UEs.

Aspect 21 is the method of any of aspects 15 to 20, wherein the method further comprises receiving a second control message from at least one of the set of UEs associated with a first periodicity lower than a second periodicity associated with the control message. The method further comprises receiving a second set of positioning signals from at least one of the set of UEs based on the second control message. The method further comprises measuring the second set of positioning signals based on the second control message. The method further comprises calculating at least one of a second set of relative ranges or a second set of relative angles between the UE and at least one of the set of UEs based on the second control message.

Aspect 22 is an apparatus for wireless communication, including: a memory; and at least one processor coupled to the memory and, based at least in part on information stored in the memory, the at least one processor is configured to implement any of aspects 1 to 21.

Aspect 23 is the apparatus of aspect 22, further including at least one of an antenna or a transceiver coupled to the at least one processor.

Aspect 24 is an apparatus for wireless communication including means for implementing any of aspects 1 to 21.

Aspect 25 is a computer-readable medium (e.g., a non-transitory computer-readable medium) storing computer executable code, where the code when executed by a processor causes the processor to implement any of aspects 1 to 21.

Embodiments of the invention can be described with reference to the following numbered clauses, with preferred features laid out in the dependent clauses:
1. An apparatus for wireless communication at a user equipment (UE), comprising:
   a memory; and
   at least one processor coupled to the memory and, based at least in part on information stored in the memory, the at least one processor is configured to:
      obtain at least one of a set of relative ranges between UE pairs of a set of UEs, a set of relative angles between the UE pairs of the set of UEs, or a set of radio frequency (RF) sensing measurements associated with a user of the set of UEs; and
      calculate an exercise state based on at least one of the set of relative ranges, the set of relative angles, or the set of RF sensing measurements.
2. The apparatus of clause 1, wherein the set of UEs comprises the UE.
3. The apparatus of clause 2, wherein, to obtain the set of relative ranges, the at least one processor is configured to:
   transmit a first set of positioning signals to a subset of the set of UEs;
   receive a second set of positioning signals from the subset of the set of UEs in response to the transmission of the first set of positioning signals;
   measure the second set of positioning signals; and
   calculate the set of relative ranges based on the measured second set of positioning signals.
4. The apparatus of clause 3, wherein, to calculate the set of relative ranges, the at least one processor is configured to:
   calculate the set of relative ranges based on a first time of arrival (ToA) associated with a first UE of the subset of the set of UEs and a second ToA associated with a second UE of the subset of the set of UEs.
5. The apparatus of clause 3, wherein, to calculate the set of relative ranges, the at least one processor is configured to:
   calculate the set of relative ranges based on a round trip time (RTT) associated with the transmission of at least one of the first set of positioning signals and the reception of at least one of the second set of positioning signals.
6. The apparatus of clause 1, wherein, to obtain the set of relative ranges, the at least one processor is configured to:
   receive a first set of positioning signals from a subset of the set of UEs;
   transmit a second set of positioning signals to the subset of the set of UEs in response to the reception of the first set of positioning signals; and
   receive the set of relative ranges from the subset of the set of UEs based on the first set of positioning signals and the second set of positioning signals.
7. The apparatus of clause 1, wherein, to obtain the set of relative angles, the at least one processor is configured to:
   receive a set of positioning signals from a subset of the set of UEs;
   measure the set of positioning signals; and
   calculate the set of relative angles based on the measured set of positioning signals.
8. The apparatus of clause 7, wherein the set of relative angles comprises an angle of arrival (AoA) associated with a positioning signal of the set of positioning signals and a corresponding UE of the subset of the set of UEs.
9. The apparatus of clause 1, wherein, to obtain the set of relative angles, the at least one processor is configured to:
   transmit a set of positioning signals to a subset of the set of UEs; and
   receive the set of relative angles from the subset of the set of UEs based on the set of positioning signals.
10. The apparatus of clause 1, wherein, to obtain the set of RF sensing measurements associated with the user of the set of UEs, the at least one processor is configured to:
   transmit a set of sensing signals;
   receive a set of reflected sensing signals based on the set of sensing signals;
   measure the set of reflected sensing signals; and
   calculate the set of RF sensing measurements based on the measured set of reflected sensing signals.
11. The apparatus of clause 1, wherein the exercise state comprises at least one of:
   a movement state associated with the user of the set of UEs; or
   a type of exercise associated with the user.
12. The apparatus of clause 11, wherein the at least one processor is further configured to:
   calculate an exercise improvement metric based on the movement state and a reference movement.
13. The apparatus of clause 1, wherein the at least one processor is further configured to:
   transmit a control message to a subset of the set of UEs, wherein, to obtain the set of relative ranges or to obtain the set of relative angles, the at least one processor is configured to obtain the set of relative ranges or obtain the set of relative angles, respectively, in response to the transmission of the control message to the subset of the set of UEs.
14. The apparatus of clause 13, wherein, to calculate the exercise state, the at least one processor is configured to calculate a movement metric associated with the user associated with the set of UEs, wherein the movement metric is greater than a threshold value, wherein the at least one processor is further configured to:
   transmit a second control message to the subset of the set of UEs associated with a first periodicity lower than a second periodicity associated with the control message.
15. The apparatus of clause 1, further comprising a transceiver coupled to the at least one processor, wherein, to obtain the set of relative angles, the at least one processor is configured to:
   transmit, via the transceiver, a set of positioning signals to a subset of the set of UEs; and
   receive, via the transceiver, the set of relative angles from the subset of the set of UEs based on the set of positioning signals.
16. An apparatus for wireless communication at a user equipment (UE), comprising:
   a memory; and
   at least one processor coupled to the memory and, based at least in part on information stored in the memory, the at least one processor is configured to:
      receive a control message from at least one of a set of UEs;
      receive a set of positioning signals from at least one of the set of UEs based on the control message;
      measure the set of positioning signals based on the control message; and
      calculate at least one of a set of relative ranges or a set of relative angles between the UE and at least one of the set of UEs based on the control message.
17. The apparatus of clause 16, wherein the control message comprises at least one of a set of resources associated with the set of positioning signals, or an indication of a set of transmission times associated with the set of positioning signals.
18. The apparatus of clause 16, wherein the at least one processor is further configured to:
   transmit at least one of the calculated set of relative ranges or the calculated set of relative angles based on the control message.
19. The apparatus of clause 16, wherein, to calculate the set of relative ranges, the at least one processor is configured to:
   calculate the set of relative ranges based on a first time of arrival (ToA) associated with a first positioning signal of the set of positioning signals from a first UE of the set of UEs and a second ToA associated with a second positioning signal of the set of positioning signals from a second UE of the set of UEs.
20. The apparatus of clause 16, wherein the at least one processor is further configured to:
   transmit a second set of positioning signals to at least one of the set of UEs based on the control message, wherein, to calculate the set of relative ranges, the at least one processor is configured to calculate the set of relative ranges based on a round trip time (RTT) associated with the transmission of at least one of the second set of positioning signals and the reception of at least one of the set of positioning signals.
21. The apparatus of clause 16, wherein the set of relative angles comprises an angle of arrival (AoA) associated with a positioning signal of the set of positioning signals and a corresponding UE of the set of UEs.
22. The apparatus of clause 16, wherein the at least one processor is further configured to:
   receive a second control message from at least one of the set of UEs associated with a first periodicity lower than a second periodicity associated with the control message;
   receive a second set of positioning signals from at least one of the set of UEs based on the second control message;
   measure the second set of positioning signals based on the second control message; and
   calculate at least one of a second set of relative ranges or a second set of relative angles between the UE and at least one of the set of UEs based on the second control message.
23. The apparatus of clause 16, further comprising a transceiver coupled to the at least one processor the at least one processor is configured to:
   receive the control message via the transceiver; and
   receive the set of positioning signals via the transceiver.
24. A method of wireless communication at a user equipment (UE), comprising:
   obtaining at least one of a set of relative ranges between UE pairs of a set of UEs, a set of relative angles between the UE pairs of the set of UEs, or a set of radio frequency (RF) sensing measurements associated with a user of the set of UEs; and
   calculating an exercise state based on at least one of the set of relative ranges, the set of relative angles, or the set of RF sensing measurements.
25. The method of clause 24, wherein obtaining the set of relative ranges comprises:
   transmitting a first set of positioning signals to a subset of the set of UEs;
   receiving a second set of positioning signals from the subset of the set of UEs in response to the transmission of the first set of positioning signals;
   measuring the second set of positioning signals; and
   calculating the set of relative ranges based on the measured second set of positioning signals.
26. The method of clause 24, wherein obtaining the set of relative ranges comprises:
   receiving a first set of positioning signals from a subset of the set of UEs;
   transmitting a second set of positioning signals to the subset of the set of UEs in response to the reception of the first set of positioning signals; and
   receiving the set of relative ranges from the subset of the set of UEs based on the first set of positioning signals and the second set of positioning signals.
27. The method of clause 24, wherein obtaining the set of RF sensing measurements associated with the user of the set of UEs, comprises:
   transmitting a set of sensing signals;
   receiving a set of reflected sensing signals based on the set of sensing signals;
   measuring the set of reflected sensing signals; and
   calculating the set of RF sensing measurements based on the measured set of reflected sensing signals.
28. A method of wireless communication at a user equipment (UE), comprising:
   receiving a control message from at least one of a set of UEs;
   receiving a set of positioning signals from at least one of the set of UEs based on the control message;
   measuring the set of positioning signals based on the control message; and
   calculating at least one of a set of relative ranges or a set of relative angles between the UE and at least one of the set of UEs based on the control message.
29. The method of clause 28, further comprising:
   transmitting at least one of the calculated set of relative ranges or the calculated set of relative angles based on the control message.
30. The method of clause 28, further comprising:
   receiving a second control message from at least one of the set of UEs associated with a first periodicity lower than a second periodicity associated with the control message;
   receiving a second set of positioning signals from at least one of the set of UEs based on the second control message;
   measuring the second set of positioning signals based on the second control message; and
   calculating at least one of a second set of relative ranges or a second set of relative angles between the UE and at least one of the set of UEs based on the second control message.

## Claims

1. An apparatus for wireless communication at a user equipment (UE), comprising:
a memory; and
at least one processor coupled to the memory and, based at least in part on information stored in the memory, the at least one processor is configured to:
receive a control message from at least one of a set of UEs;
receive a set of positioning signals from at least one of the set of UEs based on the control message;
measure the set of positioning signals based on the control message; and
calculate at least one of a set of relative ranges or a set of relative angles between the UE and at least one of the set of UEs based on the control message.

2. The apparatus of claim 1, wherein the control message comprises at least one of a set of resources associated with the set of positioning signals, or an indication of a set of transmission times associated with the set of positioning signals.

3. The apparatus of claim 1, wherein the at least one processor is further configured to:
transmit at least one of the calculated set of relative ranges or the calculated set of relative angles based on the control message.

4. The apparatus of claim 1, wherein, to calculate the set of relative ranges, the at least one processor is configured to:
calculate the set of relative ranges based on a first time of arrival (ToA) associated with a first positioning signal of the set of positioning signals from a first UE of the set of UEs and a second ToA associated with a second positioning signal of the set of positioning signals from a second UE of the set of UEs.

5. The apparatus of claim 1, wherein the at least one processor is further configured to:
transmit a second set of positioning signals to at least one of the set of UEs based on the control message, wherein, to calculate the set of relative ranges, the at least one processor is configured to calculate the set of relative ranges based on a round trip time (RTT) associated with the transmission of at least one of the second set of positioning signals and the reception of at least one of the set of positioning signals.

6. The apparatus of claim 1, wherein the set of relative angles comprises an angle of arrival (AoA) associated with a positioning signal of the set of positioning signals and a corresponding UE of the set of UEs.

7. The apparatus of claim 1, wherein the at least one processor is further configured to:
receive a second control message from at least one of the set of UEs associated with a first periodicity lower than a second periodicity associated with the control message;
receive a second set of positioning signals from at least one of the set of UEs based on the second control message;
measure the second set of positioning signals based on the second control message; and
calculate at least one of a second set of relative ranges or a second set of relative angles between the UE and at least one of the set of UEs based on the second control message.

8. The apparatus of claim 1, further comprising a transceiver coupled to the at least one processor the at least one processor is configured to:
receive the control message via the transceiver; and
receive the set of positioning signals via the transceiver.

9. A method of wireless communication at a user equipment (UE), comprising:
receiving a control message from at least one of a set of UEs;
receiving a set of positioning signals from at least one of the set of UEs based on the control message;
measuring the set of positioning signals based on the control message; and
calculating at least one of a set of relative ranges or a set of relative angles between the UE and at least one of the set of UEs based on the control message.

10. The method of claim 9, further comprising:
transmitting at least one of the calculated set of relative ranges or the calculated set of relative angles based on the control message.

11. The method of claim 9, further comprising:
receiving a second control message from at least one of the set of UEs associated with a first periodicity lower than a second periodicity associated with the control message;
receiving a second set of positioning signals from at least one of the set of UEs based on the second control message;
measuring the second set of positioning signals based on the second control message; and
calculating at least one of a second set of relative ranges or a second set of relative angles between the UE and at least one of the set of UEs based on the second control message.
